# EUROPEAN PATENT APPLICATION

(11) **EP 4 710 919 A1**
(43) Date of publication of application: **18.03.2026**
(21) Application number: 24803109.8
(22) Date of filing: 11.05.2024
(51) Int. Cl.: A61K 9/08, A61K 31/664, A61K 31/396, A61K 31/04, A61K 31/06, C07F 9/24, C07F 9/564, C07C 205/38, A61K 47/10, A61P 35/00

(54) **INJECTION PREPARATION AND PREPARATION METHOD THEREFOR, AND COMPATIBLE MEDICINAL LIQUID AND PREPARATION METHOD THEREFOR**

(30) Priority: 11.05.2023 CN 202310532733
(71) Applicant: Ascentawits Pharmaceuticals, Ltd., Shenzhen, Guangdong 518122 (CN)
(72) Inventor: DUAN, Jianxin, Shenzhen, Guangdong 518122 (CN); CAI, Xiaohong, Shenzhen, Guangdong 518122 (CN); YU, Jibing, Shenzhen, Guangdong 518122 (CN); RUAN, Meizhen, Shenzhen, Guangdong 518122 (CN); SHEN, Guangbin, Shenzhen, Guangdong 518122 (CN); HUA, Shibiao, Shenzhen, Guangdong 518122 (CN); LI, Bing, Shenzhen, Guangdong 518122 (CN); WANG, Cheng, Shenzhen, Guangdong 518122 (CN)
(74) Representative: Haseltine Lake Kempner LLP
(86) International application number: PCT/CN2024/092639
(87) International publication number: WO 2024/230831

(57) **Abstract**

An injection preparation and a preparation method therefor, and a compatible medicinal liquid and a preparation method therefor. Upon performing tests, the provided injection preparation and compatible medicinal liquid thereof meet the requirements of long-term clinical trials and commercial production and sales. The injection preparation is a solution containing a compound of the following formula I, wherein the solvent of the solution contains C2-C8 alcohol, and can also contain another cosolvent or solubilizer. The preferred injection preparation is a mixed solution of DMA, ELP and ethanol which contains 10 mg/ml of a compound of formula (I), wherein DMA is a cosolvent, and ELP is a solubilizer. For the ready-to-use injection (compatible medicinal liquid) of the compound of following formula (I), the solvent thereof is water, the solute thereof comprises the compound of formula I, DMA, ELP, ethanol, an isosmotic adjusting agent and a pH regulator, the concentration of compound A in the injection is 0.016-1.10 mg/ml, the pH of the injection is 7.4, and the injection is an isosmotic solution. Further disclosed is a method for preparing the above-mentioned injection preparation and a method for preparing the above-mentioned compatible medicinal liquid of the injection.

## Description

### Technical Field

The present invention relates to the research and development of an injection containing Compound No.16 disclosed in the patent application PCT/CN2020/089692 (with Publication No: WO2020228685A9), and belongs to the field of developing compound-based preparations for cancer therapy.

### Background Art

Compound No. 16 disclosed in the patent application No. PCT/CN2020/089692 (with Publication No. WO2020228685A9) and Compound A disclosed in the patent application No. PCT/CN2021/129077 (with Publication No. WO2023077452A1) (both applications were filed by the same applicant as the present application) refer to the same compound having a structure of the following formula (I) (hereinafter referred to as Compound A). After conducting more studies, it has been found that the compound of formula (I) has excellent anticancer efficacy, safety and stability without toxic side effects. In particular, it can specifically recognize the overexpressed aldo-keto reductase 1C3 (AKR1C3) in tumor tissues or cells and is suitable for targeted therapy. Moreover, it can penetrate into the central nervous system (such as the brain) and exert its efficacy. The compound of formula (I) (Compound A) is designated as 1-(3-((2,4'-difluoro-1,1'-biphenyl]-4-yl)oxy)-4-nitrophenyl)-2,2,2-trifluoroethyl-bis(aziridine-1-yl)phosphate, and has a structure as shown by the following formula:

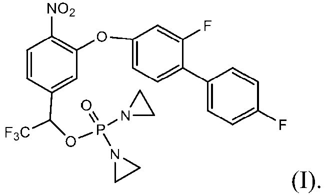

In order to perform the subsequent clinical trials, there is a need to prepare a suitable dosage form to be administered to a patient, typically by an oral or injection route. Therefore, there is a need to develop a pharmaceutical preparation for Compound A (or the compound of formula (I); also referred to as the active pharmaceutical ingredient or API in the present application) for treating cancer patients who overexpress aldo-keto reductase 1C3 (AKR1 C3).

The applicant in the patent application No. PCT/CN2020/101870 (with Publication No.: WO2021008520A1, corresponding to the Chinese patent application No.202080001484.5 with Publication No.: CN112469394A) has disclosed the formulation and preparation method of an injection preparation of Compound AST-3424 having a similar structure to Compound A. Both of the compounds are nitrobenzyl derivatives and are attached to a DNA alkylating agent having an aziridine structure that can effectively kill tumor cells. By releasing the DNA alkylating agent having an aziridine structure in tumor tissues or cells that overexpress the AKR1C3 enzyme, they can specifically target and treat the tumor tissues or cells that overexpress AKR1C3 to achieve the purpose of killing the tumor tissues or cells.

Therefore, similarly, based on the concept of developing a suitable preparation and particularly, an injection preparation for Compound AST-3424, the inventors have designed and screened out an injection preparation for the research of a formulation containing Compound A.

### Summary of the Invention

The inventors found that Compound A was poorly soluble in the mixed solvent of ethanol and propylene glycol that was used as the solvent in the formulation containing Compound AST-3424; that is, the concept of using a formulation similar to that of AST-3424 to study and screen out a liquid preparation for Compound A was unrealistic. To address this problem, the inventors have further conducted more studies, and developed the following stable preparations and preparation method applicable to Compound A.

The present invention provides a Compound A-containing injection preparation and preparation method, and also a compatible medicinal solution and the preparation method thereof. The injection preparation provided by the technical solutions of this invention has been validated in experiments as being capable of meeting the requirements of long-term clinical trials and commercial production or sales. Specifically, the present application discloses a series of technical solutions for the injection comprising Compound A.

**The present invention provides a Compound A-containing injection preparation, which is a solution comprising the compound of Formula (I):**

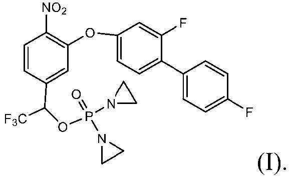

This solution is a storage-stable, concentrated Compound A-containing injection preparation, which needs to be diluted before administration, and needs to be diluted or prepared as required, when in use, by medical staff or pharmacists or staff in pharmacies or factories, to obtain a ready-to-use injection for injection use.

The concentrated injection of the present invention may be diluted with a solution formulated by using any pharmaceutically acceptable isotonic regulator (as a solute) and water for injection (as a solvent).

A suitable isotonic regulator includes, but is not limited to, anhydrous or hydrous sodium chloride, glucose, sucrose, fructose, xylitol, glycerol, sorbitol, mannitol, potassium chloride, mannose, calcium chloride, magnesium chloride, and other inorganic salts. Preferably, the isotonic regulator is glucose or sodium chloride.

**In the Compound A-containing injection preparation solution provided by the present invention, the solvent of the solution contains a C2-C8 alcohol or a liquid mixture thereof.**

The C2-C8 alcohol includes linear, branched or cyclic aliphatic alcohols and aromatic alcohols, but excluding phenols and highly active benzyl alcohol.

Specifically, the C2-C8 alcohol is a monohydric alcohol or a polyhydric alcohol. The polyhydric alcohol is a dihydric alcohol, a trihydric alcohol or a hexahydric alcohol. Preferably, the C2-C8 alcohol is a monohydric alcohol, and more preferably ethanol.

Furthermore, the C2-C8 alcohol includes C2-C4 monohydric alcohol, C2-C6 dihydric alcohol and C3-C6 trihydric alcohol. Therefore, the solvent of the above-mentioned injection preparation solution can be a liquid mixture of C2-C4 monohydric alcohol and C2-C6 dihydric alcohol, or a liquid mixture of C2-C4 monohydric alcohol and C3-C6 trihydric alcohol.

Evidently, the mixed solvent of C2-C8 monohydric alcohol and C2-C8 dihydric alcohol, trihydric alcohol or hexahydric alcohol as defined in the present invention means that each of the above-mentioned monohydric alcohol and dihydric alcohol, trihydric alcohol or hexahydric alcohol should be in the form of a liquid at normal temperature and normal pressure, or that the mixed solvent is a liquid.

Furthermore, the dihydric alcohol includes ethylene glycol and propylene glycol; the trihydric alcohol includes glycerol; and the hexahydric alcohol includes mannitol or sorbitol.

In the Compound A-containing injection preparation provided by the present invention, the monohydric alcohol contained in the solvent of the solution is preferably ethanol. As a vehicle or carrier for the active pharmaceutical ingredient Compound A in the injection, ethanol is also one of the commonly used solvents for injections in medicine. However, the inventors have found in experiments that the active pharmaceutical ingredient Compound A has low solubility in ethanol alone or in a mixed solvent of ethanol with the aforementioned polyols. Therefore, a particular surfactant is needed to be used in combination as a hydrotropic agent or solubilizer to increase the solubility of Compound A in the solvent system, and even to increase its stability.

In the Compound A-containing injection preparation solution provided by the present invention, the solvent of the solution further comprises, in addition to the C2-C8 alcohol or the liquid mixture thereof, at least one surfactant, and particularly a non-ionic surfactant. The above-mentioned surfactant has a solubilizing effect.

**Preferably, the solvent of the solution further comprises, in addition to the C2-C8 alcohol or the liquid mixture thereof, a non-ionic surfactant as a solubilizer.**

The process of increasing the solubility of poorly soluble drugs by adding a surfactant is called as solubilization, and the added surfactant is a solubilizing agent.

A surfactant refers to a substance that can significantly reduce the surface tension of the target solution. Generally, a surfactant has fixed hydrophilic and lipophilic groups that can be arranged directionally on the surface of the solution. The molecular structure of surfactants has two parts: one part contains a hydrophilic group and the other part contains a hydrophobic group. The hydrophilic group is typically a polar group, such as carboxylic acid, sulfonic acid, sulfuric acid, amino or amine group and a salt thereof. The hydroxyl group, amide group, and ether bond can also be used as a polar hydrophilic group. The hydrophobic group is typically a non-polar hydrocarbon chain, such as a hydrocarbon chain with 8 or more carbon atoms. Surfactants are classified into non-ionic surfactants and ionic surfactants according to the presence or absence of undissociated ether groups as the major hydrophilic groups in the aqueous solution.

Non-ionic surfactants refer to surfactants that do not dissociate in water, including but not limited to ester-type surfactants, Span-type surfactants, ether-type surfactants, peregal-type surfactants, amine-type surfactants, amide-type surfactants, Tween-type surfactants, and ester- and-ether-type surfactants.

The non-ionic surfactant can be a polyethylene glycol (PEG) comprising -O-CH₂-CH₂- as a repeating unit, such as polyethylene glycol 200 (PEG200), polyethylene glycol 400 (PEG400), polyethylene glycol 600 (PEG600), polyethylene glycol 800 (PEG800), and the like. Among them, PEG400 is a liquid that is high compatible with various solvents, and it is also a good solvent and solubilizer that has been widely used in liquid preparations.

The non-ionic surfactant may also be a surfactant having a polyethylene glycol (or polyoxyethylene) chain segment as the hydrophilic part. Polyethylene glycol has low toxicity and can be metabolized completely in the body. It has simple ethylene oxide repeating units that enable it to have good solubility in both organic solvents and aqueous solutions. Different types of surface active agents may be formed when the polyethylene glycol segment is attached to different hydrophobic substances, and changes in the polyethylene glycol (PEG) chain (e.g., chain length, number of chains, and attachment position) can further diversify the options and varieties of the non-ionic surfactant.

Furthermore, the non-ionic surfactant of the present invention can be selected from polyethylene glycols, hydroxystearate esters, polyoxyethylene alkyl ethers, Tweens, Spans or polyoxyethylene castor oils. Of course, a mixture of two or more surfactants mentioned above can also be selected for use, as long as the mixed surfactants can still significantly reduce the surface tension of the target solution. The non-ionic surfactant provided by the present invention can solubilize the poorly soluble substances and act as a solubilizer.

Hydroxystearic acid polyethylene glycol esters, including 15-hydroxystearic acid polyethylene glycol ester (HS15) and 12-hydroxystearic acid polyethylene glycol ester (HS12), can be obtained by dehydration condensing polyethylene glycol single chains containing different ethylene oxide repeating units with hydroxystearic acids to form an ester linkage. They are commonly used as excipients for lipophilic drugs in preparations, and mainly serve as non-ionic solubilizers.

By attaching polyethylene glycol single chains having different molecular weights to simple fatty alkyl long chains, polyoxyethylene alkyl ethers, comprising lauryl ether alkyl, oleyl ether alkyl, stearyl alcohol alkyl or hexadecyl, can be obtained. They can be used as solubilizers in preparations. Any of polyoxyethylene alkyl ethers that can be used as solubilizers can be a candidate for the non-ionic surfactant of the present application. The polyoxyethylene alkyl ethers have the following general structural formulas B and C. When n represents a different value and R represents a different group, different polyoxyethylene alkyl ethers can be obtained. 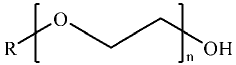
(polyoxyethylene alkyl ethers having general structural formula B),
n=23, R=CH₂(CH₂)₁₀CH₃; n=20, R=CH₂(CH₂)₁₄CH₃; or
n=20, R=CH₂(CH₂)₁₆CH₃; n=20, R={CH₂(CH₂)₁₆CH=CH(CH₂)₇CH₃}.

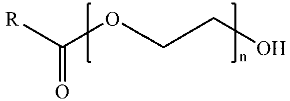

(polyoxyethylene alkyl ethers having general structural formula C),
n=8, R=CH₂(CH₂)₁₅CH₃; or n=40, R=CH₂(CH₂)₁₅CH₃.

With anhydrosorbitol in the central structure, one end for each of the four polyethylene glycol (PEG) segments is attached to the central unit, and the tail of one of the PEG segments is attached to a long fatty chain, as shown in the following formula D, which constitutes the Tween-type structural formula. Common designations of Tweens include Tween 20, Tween 40, Tween 60 and Tween 80. In these surfactants, the sum of PEG repeating units is 20; that is, w+x+y+z=20, and the fatty acid long chains attached to the tail of PEG may include lauric acid, palmitic acid, stearic acid and oleic acid. 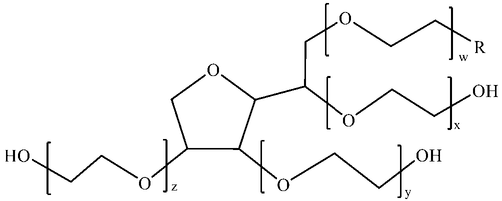
(w+x+y+z=20) (Tween surfactants having general formula D),
R=CH₂(CH₂)₉CH₃ (Tween 20); R=CH₂(CH₂)₁₃CH₃ (Tween 40);
R=CH₂(CH₂)₁₅CH₃ (Tween 60); or R={CH₂(CH₂)₆CH=CH(CH₂)₇CH₃} (Tween 80).

By removing or replacing the PEG segments in the Tween structure with long fatty chains, another type of surfactant: water-insoluble, Span-type surfactant can be obtained.

Castor oil, also known as glycerol ricinoleate, can react with ethylene oxide, such that the hydrophilic ethylene oxide structure is inserted between ricinoleic acid and glycerol to obtain polyoxyethylene castor oil. If the double bond in castor oil is hydrogenated and saturated, and then reacted with ethylene oxide, then polyoxyethylene hydrogenated castor oil would be obtained. Those skilled in the art could understand that random reaction sites of ethylene oxide and castor oil are engaged in the reaction. Thus, the obtained surfactant is a mixture that also includes unreacted castor oil and homopolymerized polyoxyethylene. Different types of polyoxyethylene castor oils or polyoxyethylene hydrogenated castor oils are determined by the ratios of ethylene oxide to castor oil or hydrogenated castor oil involved in the reaction. For example, polyoxyethylene (35) castor oil (hereinafter referred to as ELP) can be obtained by reacting 1 mol of castor oil with 35 mol of ethylene oxide.

In particular, in the technical solutions of the present invention containing surfactants, the polyethylene glycols include PEG200, PEG400, PEG600, and PEG800; the hydroxystearates include HS12 and HS15; the alkyl groups in the polyoxyethylene alkyl ethers include lauryl ether alkyl, oleyl ether alkyl, stearyl alcohol alkyl, and hexadecyl; the Tween-type surfactants include Tween 20, Tween 40, Tween 60, and Tween 80; the Span-type surfactants include Span 20, Span 40, Span 60, and Span 80; and the polyoxyethylene castor oils include polyoxyethylene castor oil and polyoxyethylene hydrogenated castor oil.

Furthermore, the polyoxyethylene castor oil of the present invention may be polyoxyethylene (35) castor oil. The pharmaceutically acceptable polyoxyethylene (35) castor oil is polyoxyethylene glycerol triricinoleate, which may also contain a small amount of polyethylene glycol ricinoleate and free ethylene glycol, and its ethylene glycol content should not exceed 620 ppm. It can be obtained by reacting 1 mol of castor oil with 35 mol of ethylene oxide. According to the gel method for bacterial endotoxin testing, as described in Volume IV, General Rule 1143 of the Chinese Pharmacopoeia (2015 Edition), the bacterial endotoxin content per 1 mg of polyoxyethylene (35) castor oil should not exceed 0.012 EU.

Preferably, in the Compound A-containing injection preparation solution provided by the present invention, the solvent of the solution further comprises, in addition to C2-C8 alcohol or the liquid mixture thereof, one or more of polyoxyethylene (35) castor oil, 15-hydroxystearate polyethylene glycol ester, polyethylene glycol 400, and Tween 80.

In the above-mentioned injection preparation solution containing a surfactant, the volume ratio of the surfactant to the C2-C8 alcohol is 1:(1-4), and in particular, the surfactant is selected from a non-ionic surfactant.

In particular, when the surfactant is ELP and the C2-C8 alcohol is ethanol, the volume ratio of ELP to ethanol is 1:3.

When the surfactant is HS15 and the C2-C8 alcohol is ethanol, the volume ratio of HS15 to ethanol is 1:4.

When the surfactant is PEG400 and the C2-C8 alcohol is ethanol, the volume ratio of PEG400 to ethanol is 1:1.

When the surfactant is Tween 80 and the C2-C8 alcohol is ethanol, the volume ratio of Tween 80 to ethanol is 1:1.

**In another Compound A-containing injection preparation solution provided by the present invention, the solvent of the solution further comprises, in addition to C2-C8 alcohol or the liquid mixture thereof, an organic solvent with a hydrotropic effect.**

This organic solvent is called as a hydrotropic agent. The poorly soluble drug and the added third substance can form a soluble composite (complex, conjugate or compound salt) in the solvent, increasing the solubility of the drug. The added third substance is also called as a hydrotropic agent. Hydrotropic agents include (1) organic acids and their salts, such as sodium benzoate, potassium iodide, sodium salicylate, and p-aminobenzoic acid; (2) amide or amine compounds, such as ethylenediamine, urethane, urea, N,N-dimethylacetamide (DMA) or N,N-dimethylformamide; and (3) water-soluble polymer compounds, such as polyvinylpyrrolidone.

Preferably, in the technical solutions of the present invention containing a hydrotropic agent, the hydrotropic agent is selected from ethylenediamine, N,N-dimethylacetamide, N,N-dimethylformamide or polyvinylpyrrolidone, and more preferably N,N-dimethylacetamide.

Pharmaceutically acceptable N,N-dimethylacetamide (DMA) should be of injectable grade with a purity of not less than 99.8%. The bacterial endotoxin content per 1 ml of N,N-dimethylacetamide should not exceed 2.0 EU as determined by the gel method in accordance with USP General Chapter <85>.

In the above-mentioned injection preparation solution containing a hydrotropic agent, the mass ratio of the organic solvent with a hydrotropic effect to compound A in the solvent of the solution is (10-50):1.

**In the Compound A-containing injection preparation solution provided by the present invention, the solvent of the solution further comprises, in addition to C2-C8 alcohol or the liquid mixture thereof, a non-ionic surfactant as a solubilizer and an organic solvent with a hydrotropic effect.**

Preferably, the non-ionic surfactant is selected from polyoxyethylene (35) castor oil, and the organic solvent with a hydrotropic effect is selected from N,N-dimethylacetamide.

In particular, the mass ratio of the non-ionic surfactant to Compound A in the solvent of the solution is (0-90):1, preferably (5-50):1, more preferably (15-40):1, even more preferably (20-40):1, and further preferably (25-40):1;

The mass ratio of the organic solvent with a hydrotropic effect in the solvent of the solution to Compound A is (0-100):1, preferably (1-50):1, more preferably (5-25):1, and further more preferably (5-10):1.

The Compound A-containing injection preparations provided by the present invention include the injection preparations in various solvents as mentioned above, wherein the solvents are selected from:
(1) C2-C8 alcohol,
(2) a combination of a C2-C8 alcohol and a non-ionic surfactant as a solubilizer,
(3) a combination of a C2-C8 alcohol and an organic solvent with a hydrotropic effect, or
(4) a combination of a C2-C8 alcohol, a non-ionic surfactant as a solubilizer, and an organic solvent with a hydrotropic effect.

Preferably, in the injection preparation provided by the present invention, the content of compound A is 0.1-200 mg/ml, preferably 1-100 mg/ml, and further preferably 10-20 mg/ml.

Considering many factors such as stability and ease of use, the Compound A-containing injection preparation provided by the present invention is a solution containing 10 mg/ml of Compound A, which is the concentration value recommended by the research and development team, and could achieve better properties in the comparison experiments.

**The present invention provides an injection preparation, comprising Compound A, N,N-dimethylacetamide, polyoxyethylene (35) castor oil and ethanol.**

**The present invention provides an injection preparation, which consists of a compound of the following formula (I), N,N-dimethylacetamide, polyoxyethylene (35) castor oil and ethanol:**

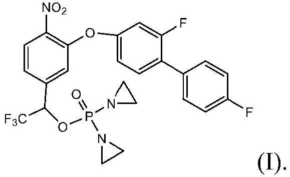

As a preferred embodiment of the present invention, the content of the compound of formula (I) is 0.1-200 mg/ml, preferably 1-100 mg/ml, more preferably 10-20 mg/ml, and even more preferably 10 mg/ml.

As a preferred embodiment of the present invention, the mass ratio of the compound of formula (I) to N,N-dimethylacetamide is 1: (0-100), preferably 1: (1-50), more preferably in the range of 1: (5-25), and further preferably in the range of 1:(5-10), and even more preferably 1:5.

As a preferred embodiment of the present invention, the mass ratio of the compound of formula (I) to polyoxyethylene (35) castor oil is 1:(0-90), preferably 1:(5-50), more preferably 1:(15-40), further preferably 1:(20-40), further preferably 1:(25-40), and even more preferably 1:40.

The present invention provides an injection preparation, which is a 2 ml ethanol solution containing 20 mg of Compound A (Formula (I)), 100 mg of N,N-dimethylacetamide and 800 mg of polyoxyethylene (35) castor oil.

The present invention provides a unit injection preparation, which is a concentrated ethanol solution labeled as having a volume of 2 ml and containing 20 mg of Compound A, 100 mg of N,N-dimethylacetamide and 800 mg of polyoxyethylene (35) castor oil.

Unit injection preparations refer to unit packaged preparations that are ultimately sold in packaging containers such as vials and ampoules. They are concentrated ethanol solutions and comprise, in addition to the drug Compound A, excipient solvents such as ethanol, hydrotropic agent DMA, and solubilizer ELP. The specification shown on the label of the unit preparation is 2 ml: 20 mg, which means that 2 ml of the unit injection preparation (i.e., concentrated ethanol solution) comprises 20 mg of the drug Compound A, 100 mg of N,N-dimethylacetamide, and 800 mg of polyoxyethylene (35) castor oil (the remainder is solvent ethanol). The values in the expressions including 2 ml: 20 mg of the drug compound A, 100 mg of N,N-dimethylacetamide and 800 mg of polyoxyethylene (35) castor oil here refer to designated values and also calculated values when charging for the formulation. The actually measured values may have certain deviations.

In order to further enhance the thermal stability of the injection preparation over time, other non-toxic substances that are non-reactive with Compound A may be added. They generally include the following substances:
Protective gases: the injection is vacuumed to significantly reduce the content of active gases such as O₂ and CO₂ contained therein, and then charged with an inert gas, such as N₂ or other inert gases, that does not react with the active pharmaceutical ingredient, monohydric alcohol, dihydric alcohol, trihydric alcohol, hexahydric alcohol, N,N-dimethylacetamide, N,N-dimethylformamide or surfactant (such as polyoxyethylene (35) castor oil, HS15 and Tween 80). The protective gas(es) will be dissolved in the injection or fill up in the packaging container.

Antioxidants: an appropriate amount of an antioxidant (such as butylated hydroxyanisole (BHA), 2,6-di-tert-butylated hydroxytoluene (BHT), vitamin E (tocopherol), vitamin C, or glutathione) is added to the injection to further improve the stability.

In order to improve the compliance of the patients, drugs having anesthetic or analgesic effects may be further added in allowable doses that are prescribed in the Pharmacopoeia or Formulary accordingly.

The injection disclosed in the present invention may also contain an additional therapeutic agent to form a compound preparation that can exhibit a synergistic effect to enhance the therapeutic effect. In particular, it is recommended that these drugs are those capable of adjusting the content of the AKR1 C3 enzyme or the expression level of the corresponding gene.

In some situations, in order to accommodate special environments, the injection disclosed in the present invention may also be added with an antibacterial or antifungal agent.

In addition, substances such as electrolytes (such as salts including NaCl and KCl), glucose and amino acids that are frequently used in injections to regulate the acid-base balance and the osmotic pressure may also be added as appropriate.

Apparently, the above-mentioned protective gases, antioxidants, drugs with anesthetic or analgesic effects, antibacterial agents, antifungal agents and the like are added in very small amounts, and would not affect the properties such as solubility of Compound A, or could improve the stability. The additional therapeutic agent and the substances for regulating the acid-base balance and the osmotic pressure need to be added according to the actual situations, e.g., purposes, prescribed amounts, and instructions in the Pharmacopoeia and Formulary.

The Compound A-containing injection preparation provided by the present invention is not added with water, and the content of water by mass is less than or equal to 1.0%, and preferably less than or equal to 0.5%.

Evidently, no addition of water means that no water is additionally charged in the preparation process, and the other reagents, solvents, and the like used are anhydrous reagents such as anhydrous ethanol, anhydrous propylene glycol, anhydrous polyoxyethylene (35) castor oil and anhydrous N,N-dimethylacetamide. In other words, the water content is required to be controlled during the preparation process of the injection of the present invention. In theory, the lower the water content of the injection is, the better the stability will be. In view of the technical difficulties and the actual conditions of industrial mass production, the research and development team has concluded that the water content (determined by the Karl Fischer method) should be within 1.0% by mass, and preferably no more than 0.5% by mass.

The inventors have found through research that Compound A has a gradually increasing solubility in ethanol, polyoxyethylene (35) castor oil and N,N-dimethylacetamide, respectively; that is, the Compound A has the lowest solubility in ethanol and the highest solubility in N,N-dimethylacetamide. Therefore, the present invention also provides technical solutions involving the following injection preparations:
i) An injection preparation, comprising Compound A and polyoxyethylene (35) castor oil, with or without ethanol;
ii) An injection preparation, comprising Compound A and N,N-dimethylacetamide, with or without ethanol; and
iii) An injection preparation, comprising Compound A, polyoxyethylene (35) castor oil and N,N-dimethylacetamide, without ethanol.

Obviously, when ethanol is not contained, the injection preparation i) is a solution comprising polyoxyethylene (35) castor oil as a solvent; the injection preparation ii) is a solution comprising N,N-dimethylacetamide as a solvent; and the injection preparation iii) can be considered as a solution comprising N,N-dimethylacetamide as a solvent. Those skilled in the art could understand that the above-mentioned injection preparations i), ii) and iii) are injection solutions in liquid form comprising Compound A as the active pharmaceutical ingredient.

Of course, in order to further enhance the thermal stability of the injection preparations i), ii) and iii) over time or improve compliance of patients, other substances that are non-toxic and non-reactive with Compound A may be added, such as the protective gas, antioxidants and drugs with anesthetic or analgesic effects as mentioned above.

The injections i), ii) and iii) disclosed in the present invention may also contain an additional therapeutic agent to form a compound preparation that could exhibit a synergistic effect to enhance the therapeutic effect. In particular, it is recommended that these drugs are those capable of adjusting the content of the AKR1C3 enzyme or the expression level of the corresponding gene.

In some situations, in order to accommodate special environments, the injections i), ii) and iii) disclosed in the present invention may also be added with an antibacterial or antifungal agent.

In addition, substances such as electrolytes (such as salts including NaCl and KCl), glucose and amino acids that are frequently used in the injection to regulate the acid-base balance and the osmotic pressure may also be added as appropriate.

Apparently, the above-mentioned protective gases, antioxidants, drugs with anesthetic or analgesic effects, antibacterial agents, antifungal agents and the like are added in very small amounts, and would not affect the properties such as solubility of Compound A, or could improve the stability. The additional therapeutic agent and the substances for regulating the acid-base balance and the osmotic pressure need to be added according to the actual situations, e.g., purposes, prescribed amounts, and instructions in the Pharmacopoeia and Formulary.

The injection preparations i), ii) and iii) provided by the present invention is not added with water, and the mass content of water is less than or equal to 1.0%, and preferably less than or equal to 0.5%.

**The present invention provides an injection preparation product, comprising a packaging container and an injection preparation contained in the container, wherein the injection preparation is any one of the injection preparations mentioned above and is filled in a lightproof glass vial made of a neutral borosilicate glass tube; wherein the content of the compound of formula (I) in the injection preparation is 0.1-200 mg/ml, preferably 1-200 mg/ml, and further preferably 10-20 mg/ml.**

The present invention provides an injection preparation product, which essentially consists of Compound A, N,N-dimethylacetamide, polyoxyethylene (35) castor oil and ethanol, and the injection preparation is filled in a 2 ml-, 5 ml-, or 10 ml-lightproof glass vial made of a neutral borosilicate glass tube; in particular, the content of Compound A in the injection preparation is 10 mg/ml, the mass ratio of the compound of formula (I) to N,N-dimethylacetamide is 1:5, and the mass ratio of the compound of formula (I) to polyoxyethylene (35) castor oil is 1:40.

A specific example of the Compound A-containing injection preparation is as mentioned above. The injection preparation can be filled in a 2 ml-, 5 ml-, or 10 ml-lightproof glass vial made of a neutral borosilicate glass tube, and then capped with a rubber stopper and sealed with an aluminum-plastic composite cap for antibiotic vial. Obviously, the lightproof glass vial and the rubber stopper are the inner packaging materials that can come into direct contact with the injection preparation.

In particular, the concentration of Compound A as the active pharmaceutical ingredient in the injection of this example will be calibrated to 10 mg/ml; or the concentration of N,N-dimethylacetamide will be calibrated to 50 mg/ml; or the concentration of polyoxyethylene (35) castor oil will be calibrated to 400 mg/ml. In the criteria for pharmaceuticals, the actually measured content may also change, and a concentration should be considered to be acceptable as long as it is within the corresponding range prescribed by the Pharmacopoeia or Formulary or criteria for pharmaceuticals. In other words, the concentration within the above-mentioned corresponding range is technically equivalent to the calibrated concentration.

Similarly, during the preparation or mixing process of the solution, the actual volume measured after mixing is often smaller than the sum of the volumes before mixing. Therefore, in the technical solutions provided by the present invention, the reasonable volume changes caused by the preparation or mixing of the solution are also well known to those skilled in the art (researchers or medical staff in the fields including the research and development of pharmaceuticals, organic chemical synthesis, and research and development of formulations).

The present invention provides a method for preparing an injection preparation, comprising the following steps: adding a prescribed amount of Compound A to a prescribed amount of N,N-dimethylacetamide under stirring until dissolution, then adding a prescribed amount of polyoxyethylene (35) castor oil under stirring until dissolution, and finally adding ethanol until the prescribed volume for mixing and dissolving to obtain the injection preparation.

Furthermore, the method for preparing the injection preparation comprises adding 20 mg of Compound A to 100 mg of N,N-dimethylacetamide under stirring until dissolution, then adding 800 mg of polyoxyethylene (35) castor oil under stirring until dissolution, and finally adding ethanol until a volume of 2ml for mixing and dissolving.

The present invention further provides another method for preparing an injection preparation, comprising the following steps: adding a prescribed amount of Compound A to a prescribed amount of N,N-dimethylacetamide under stirring until dissolution to obtain a first-dissolution preparation liquor; adding a prescribed amount of polyoxyethylene (35) castor oil to a portion of ethanol under stirring until dissolution to obtain a second-dissolution preparation liquor; and adding the first-dissolution preparation liquor to the second-dissolution preparation liquor under stirring until the mixed preparation is clear, and then adding ethanol until the prescribed volume for mixing and dissolving to obtain the injection preparation.

This operation is contemplated in view of the fact that polyoxyethylene (35) castor oil is relatively viscous below 20°C. Therefore, polyoxyethylene (35) castor oil and part of anhydrous ethanol are initially mixed uniformly.

The present invention further provides an injection for the injection of Compound A, which is a ready-to-use injection of a pharmaceutical composition. This composition does not need to be diluted before administration. This composition has already been suitable for administration once it is produced, and does not require the medical staff to conduct dilution or preparatory work prior to use.

Obviously, the injection for the injection of Compound A may be a ready-to-use injection suitable to be administered via various routes, e.g., intradermal, subcutaneous, intramuscular, and intravenous injection.

A ready-to-use injection for intravenous injection is described below as an example. Those skilled in the art could also design other intradermal, subcutaneous, or intramuscular (muscular) injections according to clinical needs.

The ready-to-use injection for the injection of Compound A provided by the present invention is prepared from water, an isotonic regulator, and any one of the injection preparations as mentioned above, wherein the injection is added with or not added with a pH regulator.

The pH regulator, i.e., the pH-regulating substance, includes alkali metal salts or alkaline earth metal salts of weak acids such as carbonic acid, phosphoric acid, citric acid, and acetic acid, and inorganic bases such as hydroxides of alkali metals or alkaline earth metals such as Na and K, for example, sodium citrate, potassium citrate, sodium acetate, potassium acetate, potassium dihydrogen phosphate, sodium dihydrogen phosphate, dipotassium hydrogen phosphate, disodium hydrogen phosphate, potassium phosphate, sodium phosphate, sodium hydroxide, potassium hydroxide, sodium bicarbonate, potassium bicarbonate, sodium carbonate, and potassium carbonate.

The present invention provides an example of a ready-to-use injection for the injection of Compound A, which contains water, an isotonic regulator, Compound A, N,N-dimethylacetamide, polyoxyethylene (35) castor oil, ethanol, and a pH adjuster.

Obviously, the ready-to-use injection as mentioned above is an isotonic solution; the pH value is ranging from 6.8 to 10.5; and the concentration of Compound A is 0.016-1.10 mg/ml.

A solution with a concentration of 308 mmol/L is generally considered to be an isotonic solution for humans, such as 0.9% normal saline and 5% glucose solution. Of course, in a less strict sense, a solution having a concentration between 280 and 320 mmol/L is also considered as an isotonic solution.

Of course, if the subjects to be treated are other animals, such as other primates, the concentration of the isotonic solution should be adjusted accordingly.

In particular, the concentration of Compound A is 0.02-1.00 mg/ml.

In particular, the pH value of the ready-to-use injection for injection use is 7.4-8.1, and preferably 7.7. The injection for injection use with a pH within the above-mentioned pH range is more stable, and can ensure storage for a certain period of time (for example, after on-site preparation, the patient cannot receive the injection on time temporarily, or during intravenous drip, the drip process may also take a certain amount of time to finish the injection).

The pH regulator used in the injection for the injection of Compound A as provided by the present invention, i.e., the pH-regulating substance as mentioned above, includes alkali metal salts or alkaline earth metal salts of weak acids such as carbonic acid, phosphoric acid, citric acid, and acetic acid, and inorganic bases such as hydroxides of alkali metals or alkaline earth metals such as Na and K, for example, sodium citrate, potassium citrate, sodium acetate, potassium acetate, potassium dihydrogen phosphate, sodium dihydrogen phosphate, dipotassium hydrogen phosphate, disodium hydrogen phosphate, potassium phosphate, sodium phosphate, sodium hydroxide, potassium hydroxide, sodium bicarbonate, potassium bicarbonate, sodium carbonate, and potassium carbonate. Obviously, the pH regulator included in the ready-to-use injection of the present invention can be a single substance as mentioned above, or a combination of multiple substances that can have a synergistic effect in regulating the pH value.

The isotonic regulator included in the ready-to-use injection for the injection of Compound A provided by the present invention can be any pharmaceutically acceptable osmotic pressure regulating agent for the injection of the present invention. Suitable osmotic pressure regulator includes, but is not limited to, anhydrous or hydrous sodium chloride, glucose, sucrose, fructose, xylitol, glycerol, sorbitol, mannitol, potassium chloride, mannose, calcium chloride, magnesium chloride, other inorganic salts, or a mixture thereof.

The present invention provides a ready-to-use injection for the injection of Compound A, which consists of water, glucose as an isotonic regulator, Compound A, N,N-dimethylacetamide, polyoxyethylene (35) castor oil, ethanol, and sodium bicarbonate as a pH regulator. The injection is an isotonic solution with a pH value of 7.4, a concentration of Compound A of 0.016-1.10 mg/ml, and a glucose content of 4.5-5.0% by mass.

Preferably, the concentration of the Compound A is 0.02-1.00 mg/ml.

Preferably, the mass ratio of the Compound A to N,N-dimethylacetamide is 1:5.

Preferably, the mass ratio of the Compound A to polyoxyethylene (35) castor oil is 1:40.

The present invention further provides a method for preparing a ready-to-use injection for the intravenous injection of Compound A, comprising the following steps:
(a) adding an appropriate amount of sodium bicarbonate solution as a pH regulator to a 5% glucose injection to obtain a mixed solution, such that the pH of the mixed solution is 7.7-8.1; and
(b) then adding any one of the injection preparations according to the present invention or an injection preparation included in any one of the injection preparation products according to the present invention to the mixed solution obtained in step (a) for mixing and dissolving to obtain the ready-to-use injection for intravenous injection.

Preferably, in step (a), the mixed solution is obtained by adding 1 volume of 5% sodium bicarbonate injection to 250 volumes of 5% glucose injection.

In a specific example, the amount and concentration of the sodium bicarbonate solution used as the pH regulator, the amount of the 5% glucose injection, and the amount of Compound A-containing injection preparation, are obtained by calculation.

### Brief description of the drawings

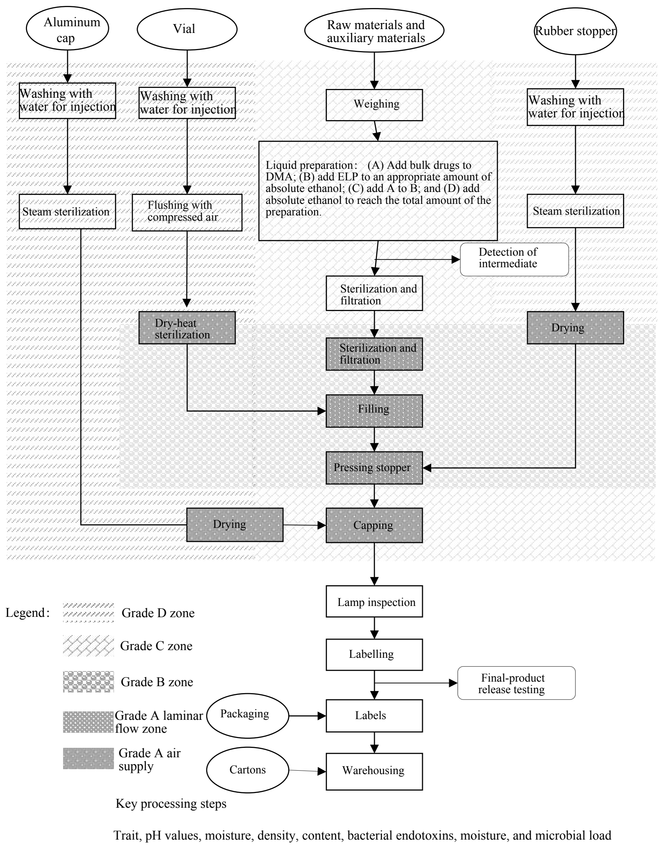

FIG. 1 is a flow chart of the production process for the concentrated Compound A-containing injection preparation.

### Detailed Description of the Invention

The non-ready-to-use concentrated injection, its packaged product and injection (ready-to-use pharmaceutical composition injection) provided by the present invention can meet the requirements for the storage stability of the compound of formula (I) (Compound A).

In certain embodiments, the concentrated injections provided by the present invention, tend to have stable results in various parameters (including trait, pH value, moisture, related substances, and content) under the conditions of long-term stability experiments (-20°C ± 5°C) for 6 months, without showing significant changes, and could meet the proposed quality standards.

In certain embodiments, the concentrated injections provided by the present invention, have single-impurity and total-impurities results that could meet the requirements of the criteria under the conditions of the accelerated stability experiments (5°C ± 3°C) for 6 months, tend to have stable results in various parameters (including trait, pH value, moisture, and content), without showing significant changes, and could meet the proposed quality standards.

In certain embodiments, the concentrated injections provided by the present invention show no obvious change in appearance after having been placed under higher temperature (25°C ± 2°C) and light exposure conditions for 30 days or placed for 3 cycles under two freeze-thaw conditions, and remain as a light-yellow clear liquid.

In certain embodiments, the concentrated injections provided by the present invention show no obvious change in pH values after having been placed under higher temperature (25°C ± 2°C) and light exposure conditions for 30 days or placed for 3 cycles under two freeze-thaw conditions and have pH values within the range of from 4.5 to 7.0.

In certain embodiments, the concentrated injections provided by the present invention have minor visible foreign matters that could meet the requirements of the criteria after having been placed under higher temperature (25°C ± 2°C) and light exposure conditions for 30 days or placed for 3 cycles under two freeze-thaw conditions.

In certain embodiments, the concentrated injections provided by the present invention have a total-impurities content less than the acceptable standard level of 5%, after having been placed under higher temperature conditions (25°C ± 2°C) for 30 days, and have a total-impurities content less than the acceptable standard level of 5% after having been placed under light exposure conditions (with outer packaging, with the illuminance level as prescribed in the Pharmacopoeia at 15°C) or light exposure conditions (without outer packaging, and with the illuminance level as prescribed in the Pharmacopoeia at 15°C) for 30 days. The increased impurity content after the light exposure with outer packaging is the same as that after the light exposure without outer packaging, and both of the increased contents are lower than that under the higher temperature conditions (25°C ± 2°C). They all could meet the requirements of the quality standards.

In certain embodiments, the concentrated injections provided by the present invention have a moisture content of less than 0.3% that could meet the requirements of the criteria after having been placed under higher temperature (25°C ± 2°C) and light exposure conditions for 30 days, or placed for 3 cycles under two freeze-thaw conditions.

In certain embodiments, the concentrated injections provided by the present invention show no growth of bacteria until the third cycle during storage for 3 cycles under two freeze-thaw conditions, and could meet the requirements of the criteria.

In certain embodiments, the concentrated injections provided by the present invention have a bacterial endotoxin content of less than 1.2 EU/mg at the third cycle during storage for 3 cycles under two freeze-thaw conditions, and could meet the requirements of the criteria.

In certain embodiments, the contents of the concentrated injections provided by the present invention are decreased by 3.4% after having been placed under higher temperature conditions (25°C ± 2°C) for 30 days and could meet the requirements of the quality standard; and no significant content changes are observed after having been placed under light exposure conditions (with outer packaging, and with the illuminance level as prescribed in the Pharmacopoeia at 15°C) and light exposure conditions (without outer packaging, and with the illuminance level as prescribed in the Pharmacopoeia at 15°C) for 30 days, or placed for 3 cycles under freeze-thaw condition 1 (-20°C ± 5°C for 2 days → 5°C ± 3°C for 2 days) and freeze-thaw condition 2 (-20°C ± 5°C for 2 days → 25°C ± 2°C for 2 days). They could all meet the requirements of the quality standards.

In certain embodiments, the ready-to-use injection provided by the present invention could remain stable in appearance, solution color, solution clarity, pH value, osmotic pressure, related substances, and content for 6 hours or even 8 hours under indoor natural light exposure and room temperature (25±3°C) storage conditions, and could meet the requirements of injection in clinical trials.

In certain embodiments, Compound A in the concentrated injection provided by the present invention and the compounding solution is chirally stable, and the two isomers would not possibly undergo chiral conversion.

The pH value of the ready-to-use injection of the present invention may be adjusted with an appropriate amount of a pH regulator containing acidic or basic groups. A suitable pH regulator generally includes at least one acid or a salt thereof, or includes at least one base or a salt thereof. An acid or base is added for adjustment to achieve the desired pH value. For example, if the pH value is lower than the desired pH value, a base (or some salts) will be added to raise the pH to the desired value.

In some examples, the concentrated injection of the present invention is sterile, for example, being sterilized by a terminal.

The composition injection of the present invention is packaged in a pharmaceutically acceptable packaging container. The packaging container may be an intravenous infusion bag or bottle. The infusion bag and bottle may be made of glass, suitable plastics, or polymer materials. The entire packaging container or most part of the packaging container may comprise the following materials: polyvinyl chloride, polyolefin, polyester, polypropylene, or a combination thereof. In other examples, only the surface material in contact with the pharmaceutical injection contains these materials. In the present invention, a penicillin vial is preferred.

The concentrated injection of the present invention may further comprise a certain amount of opioid analgesics.

The opioid analgesics may be selected from drugs such as Alfentanil, Allylrotidine, Alphaprodine, Anileridine, Apomorphine, Apocodeine, Benzmorphine, Bezitramide, Brifentanil, Buprenorphine, Butorphanol, Carfentanil, Dextromoramide, Codeine, Cyclopolychlorinated Biphenyls, Cyprenorphine, Desomorphine, Dezocine, Diampromide, Dihydrocodeine, Dimenoxadol, Dimepheptanol, Dimethylthiambutene, Dioxophenylbutyric Acid, Dipipanone, Eptazocine, Ethylmethylthiambutene, Ethylmorphine, Etonitazene, Fentanyl, Heroin, Hydrocodone, Hydroxymethylmorphine, Hydromorphone, Ketobemidone, Isomethadone, Levallorphan, Levorphanol, Levophenacylmorphan, Lofentanil, Demerol, Meptazinol, Metazocine, Methadone, Methylmorphine, Metopon, Mirfentanil, Morphine, Morphin-6-Glucuronic acid, Myrophine, Nalbuphine, Papaverine, Nicorandil, Norlevorphanol, Normethadone, Nalorphine, Orphanin/Orphanin FQ (N/OFQ), Normorphine, Norpipanone, Ohmefentanyl, Opium, Oxycodone, Oxydihydromorphinone, Papaveretum, Pentazocine, Phenadoxone, Phenomorphan, Phenazocine, Phenoperidine, Oxyhydromorphinone, Piminodine, Piritramide, Proheptazine, Promedol, Profadol, Properidine, Propiram, Propoxyphene, Remifentanil, Sufentanil, Tapentadol, Tramadol, Trefentanil, Tilidine, and Nalbuphine; any opioid that has an agonistic activity on opioid receptors and belongs to phenanthrene, morphinan, phenylmorphines, methadone, phenylpiperidine, propionanilide-4-anilinopiperidine, 4-arylpiperidine, and 4-isoarylpiperidine; any opioid that has an agonistic activity on opioid receptors and has the same pentacyclic skeleton as Nalmefene, Naltrexone, Buprenorphine, Levorphanol, Meptazinol, Pentazocine, and Dezocine; any analog of fentanyl, a prodrug, a derivative or a pharmaceutically acceptable salt thereof, or a mixture of racemate or enantiomer thereof that is active on opioid receptors.

The examples below are provided to facilitate the understanding of the present invention, but should not be construed as limitations on the present invention. For those of ordinary skill in the art, according to the idea of the present invention, there will be changes in the specific embodiments and the scope of application. The content of the present specification should not be construed as a limitation on the present invention. Any change made in accordance with the spirit of the present invention will be within the scope of protection of the present invention.

The specific tests and Examples of the present application are described below.

The following tests will reveal some physical and chemical properties of the compound of formula I (Compound A) developed by the applicant, including its solubility, formulation, compatibility, and stability. The applicant hereby declares that the rights of the following experimental data belongs to the applicant.

The compound of formula I (Compound A) has the following structure:

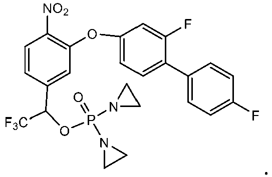

### I. Studies on Solubility and Solution Stability of Compound A (Active Pharmaceutical Ingredient or API)

### 1.1 Investigation of Solubility of Compound A in Different Solvents

The solubility of Compound A in single solvents (water, acidic and alkaline aqueous solutions, isopropanol, and methanol) was investigated. The test results are shown in Table 1.

**Table 1: Solubility investigation of Compound A at 25°C**

| Solvents (25°C) | Solubility |
|---|---|
| Water | Practically insoluble |
| 0.1 mol/L HCl solution | Practically insoluble |
| 0.1 mol/L NaOH solution | Practically insoluble |
| Methanol | Sparingly soluble |
| Isopropanol | Practically insoluble |

The above results show that Compound A has extremely low solubility in water, and acidic and alkaline aqueous solutions. Therefore, dissolution studies were made in non-aqueous solvents to screen for optimal solvents or hydrotropic agents.

In order to increase the concentration of the active pharmaceutical ingredient in the injection, the solubility of the active pharmaceutical ingredient in single adjuvants was investigated to screen for optimal solvents, hydrotropic agents, or solubilizers. The solubility test of Compound A in single solvents was conducted as follows:
Each of the single solvents was taken, a certain amount of API (the compound of formula I) was added at 25°C and stirred, and the dissolution of the API in the solvent system was observed. If the dissolution was complete, additional API was added continuously until a supersaturated solution was obtained. Samples were taken every 2 hours, filtered and assayed for content. The solubility in all single solvents at 25°C is shown in Table 2.

**Table 2: Solubility data of Compound A in single solvents at 25°C**

| Solvents | Solubility at 25°C (mg/ml)/ sampling time points (h) | | | |
|---|---|---|---|---|
| | 0h | 2h | 4h | 6h |
| N,N-Dimethylacetamide (DMA) | 248.07 | 251.41 | 251.00 | 250.88 |
| Polyoxyethylene (35) castor oil (ELP) | 18.60 | 20.70 | 20.73 | 21.29 |
| Polyethylene glycol 400 (PEG 400) | 12.21 | 11.21 | 15.34 | 15.98 |
| Ethanol | 8.95 | 9.09 | 9.43 | 9.28 |

The above data indicate that Compound A has the highest solubility (greater than 200 mg/ml) in N,N-dimethylacetamide, and has a solubility of approximately 20 mg/ml in polyoxyethylene (35) castor oil, and its solubility in polyethylene glycol 400 is slightly higher than that in absolute ethanol.

When designing injection formulations, it is necessary to add appropriate adjuvants. These adjuvants should not affect the therapeutic efficacy of the drug, must avoid interference with testing, and should be used at concentrations that do not cause toxicity or obvious irritation. In order to screen for optimal solvents, hydrotropic agents, or solubilizers, the solubility of Compound A in different adjuvants was investigated separately.

Compound A and Compound AST-3424 are structurally similar (both are nitrobenzyl derivatives and linked to a DNA alkylating agent with an aziridine structure that can effectively kill tumor cells). In addition, by releasing the DNA alkylating agent with an aziridine structure in tumor tissues or cells that overexpress the AKR1C3 enzyme, both compounds enable targeted recognition and treatment of any of the tumor tissues or cells that overexpress aldehyde-keto reductase 1C3 (AKR1C3), and achieve the purpose of killing the tumor tissues or cells. Therefore, based on the same formulation development strategy, the inventors designed and selected an injection formulation similar to that of Compound AST-3424 (the optimal formulation of AST-3424: 10 mg/ml Compound AST-3424 + 75% ethanol + 25% propylene glycol) for use in the formulation study of Compound A.

In addition to the solvents used in the reference formulation of Compound AST-3424, absolute ethanol was employed as a commonly used and relatively safe non-aqueous solvent adjuvant for injection formulations. During the development of the product formulation, it was considered to add N,N-dimethylacetamide (DMA) or polyoxyethylene (35) castor oil (ELP) to improve solubility and facilitate the manufacturing convenience. The formulation compositions with different adjuvants and solubility investigation results of Compound A are shown in Table 3.

**Table 3: Investigation on the solubility of Compound A in different adjuvants and the experimental observations before and after treatment**

| **Formulations before treatment** | **Formulation compositions** | **Preparation methods** | **Experimental observations/ treatment** |
|---|---|---|---|
| F1 | 300 mg API, absolute ethanol added to 30 ml | Approximately 12 g of absolute ethanol was added, then 300 mg API was added and mixed, and absolute ethanol was added to 30 ml | After stirring at room temperature for 4 hours, the API in none of the formulations was dissolved completely. After adding a total of 7.5 g of ELP, the API in all formulations was dissolved. |
| F2-1 | 3 g propylene glycol, 300 mg API, absolute ethanol added to 30 ml | Approximately 12 g of absolute ethanol was added and mixed with 3 g of propylene glycol, then 300 mg API was added and mixed, and absolute ethanol was added to 30 ml | |
| F2-2 | 9 g propylene glycol, 300 mg API, absolute ethanol added to 30 ml | Approximately 12 g of absolute ethanol was added and mixed with 9 g of propylene glycol, then 300 mg API was added and mixed, and absolute ethanol was added to 30 ml | |
| F3-1 | 1.5 g glycerol, 300 mg API, absolute ethanol added to 30 ml | Approximately 12 g of absolute ethanol was added and mixed with 1.5 g of glycerin, then 300 mg API was added and mixed, and absolute ethanol was added to 30 ml | After stirring at room temperature for 3 hours, the API in none of the formulations was dissolved completely. After adding a total of 7.5 g of ELP, the API in all formulations was dissolved. |
| F3-2 | 4.5 g glycerol, 300 mg API, absolute ethanol added to 30 ml | Approximately 12 g of absolute ethanol was added and mixed with 4.5 g of glycerin, then 300 mg API was added and mixed, and absolute ethanol was added to 30 ml | |
| F4-1 | 3 g DMA, 300 mg API, absolute ethanol added to 30 ml | Approximately 12 g of absolute ethanol was added and mixed with 3 g of DMA, then 300 mg API was added and mixed, and absolute ethanol was added to 30 ml | With stirring, the API in all formulations was dissolved completely within 1 hour. |
| F4-2 | 9 g DMA, 300 mg API, absolute ethanol added to 30 ml | Approximately 12 g of absolute ethanol was added and mixed with 9 g of DMA, then 300 mg API was added and mixed, and absolute ethanol was added to 30 ml | |
| F4-3 | 15 g DMA, 300 mg API, absolute ethanol added to 30 ml | Approximately 12 g of absolute ethanol was added and mixed with 15 g of DMA, then 300 mg API was added and mixed, and absolute ethanol was added to 30 ml | |

| | | | |
|---|---|---|---|
| Note: F1 refers to the group with only absolute ethanol added; the other groups include additional adjuvants/solvents as well as absolute ethanol, wherein F2 groups include propylene glycol, F3 groups include glycerin, and F4 groups include DMA. | | | |

Following the formulation design of Compound AST-3424 (the volume ratio of ethanol to propylene glycol is 3:1), a 30 ml formulation solution comprising 300 mg of Compound A was prepared, with the amount of propylene glycol (approximately 7.8 g) falling between the amounts of propylene glycol used in Formulations F2-1 and F2-2. Nevertheless, the above experimental observations indicate that Compound A cannot be completely dissolved in the formulation prepared based on the formulation design of Compound AST-3424. Therefore, the approach of preparing a formulation of the compound of formula I (Compound A) based on the formulation of Compound AST-3424 is not feasible. The inventors needed to develop other formulations for Compound A.

After treating the aforementioned formulations, the treated formulations were obtained. From the observations before and after treatment, it can be seen that the surfactant polyoxyethylene (35) castor oil (ELP) and the hydrotropic agent N,N-dimethylacetamide (DMA) contributed to the dissolution of the API. In the subsequent studies, absolute ethanol and the hydrotropic agent DMA continued to be used; and since surfactants can improve the solubility of the API in absolute ethanol, a preliminary study on different surfactants were conducted.

### 1.2 Investigation on Stability of Compound A in Different Solvent Systems

For the aforementioned treated Formulations F1 to F4, their specific formulation information is as follows ("p": the formulation obtained by adding ELP to the formulations in Table 3):
F1p: 300 mg API, 7.5 g ELP, and absolute ethanol added to 30 ml;
F2-1p: 3 g propylene glycol, 300 mg API, 7.5 g ELP, and absolute ethanol added to 30 ml;
F2-2p: 9 g propylene glycol, 300 mg API, 7.5 g ELP, and absolute ethanol added to 30 ml;
F3-1p: 1.5 g glycerin, 300 mg API, 7.5 g ELP, and absolute ethanol added to 30 ml;
F3-2p: 4.5 g glycerin, 300 mg API, 7.5 g ELP, and absolute ethanol added to 30 ml;
F4-1: 3 g DMA, 300 mg API, and absolute ethanol added to 30 ml;
F4-2: 9 g DMA, 300 mg API, and absolute ethanol added to 30 ml;
F4-3: 15 g DMA, 300 mg API, and absolute ethanol added to 30 ml.

The stability of Compound A in the aforementioned formulations was investigated. At the predetermined time points (Day 0, Day 5, Day 10), 1 ml samples were taken for HPLC analysis to detect the content, total impurities, and moisture, respectively. The different investigation conditions and experimental results are shown in Table 4.

**Table 4: Stability of Compound A in formulations with different adjuvants at 2-8°C**

| Formulations (after treatment) | Storage Conditions | Storage Time | Content (%) | Total Impurities (%) | Moisture (%) |
|---|---|---|---|---|---|
| Flp | NA | 0 day | 103.7 | 3.06 | 0.18 |
| | 2-8°C | 5 days | 99.0 | 3.05 | 0.21 |
| | | 10 days | 93.6 | 3.01 | 0.25 |
| F2-1p | NA | 0 day | 99.0 | 3.12 | 0.18 |
| | 2-8°C | 5 days | 99.0 | 3.08 | 0.22 |
| | | 10 days | 97.9 | 3.06 | 0.25 |
| F2-2p | NA | 0 day | 100.1 | 3.13 | 0.19 |
| | 2-8°C | 5 days | 97.3 | 3.09 | 0.22 |
| | | 10 days | 94.1 | 3.08 | 0.28 |
| F3-1p | NA | 0 day | 99.4 | 2.92 | 0.25 |
| | 2-8°C | 5 days | 98.8 | 2.91 | 0.29 |
| | | 10 days | 98.7 | 2.87 | 0.36 |
| F3-2p | NA | 0 day | 106.5 | 2.88 | 0.22 |
| | 2-8°C | 5 days | 92.5 | 2.87 | 0.25 |
| | | 10 days | 91.2 | 2.87 | 0.30 |
| F4-1 | NA | 0 day | 104.9 | 2.93 | 0.10 |
| | 2-8°C | 5 days | 94.9 | 2.92 | 0.14 |
| | | 10 days | 101.0 | 2.93 | 0.21 |
| F4-2 | NA | 0 day | 96.6 | 2.96 | 0.14 |
| | 2-8°C | 5 days | 94.2 | 2.95 | 0.16 |
| | | 10 days | 96.1 | 3.01 | 0.20 |
| F4-3 | NA | 0 day | 108.7 | 3.09 | 0.49 |
| | 2-8°C | 5 days | 95.3 | 3.03 | 0.52 |
| | | 10 days | 97.5 | 3.08 | 0.57 |

| | | | | | |
|---|---|---|---|---|---|
| Note: NA indicates that storage conditions are not applicable to 0 day. | | | | | |

The above data show that, when stored at 2-8°C for 10 days, Compound A remained relatively stable in all formulation solvent systems with respect to content, total impurities, and moisture.

### II. Formulation Design, Preparation and Stability Study of Compound A Injection

Based on the properties of Compound A revealed by the above solubility and solution stability studies, formulation design, preparation and stability studies were further carried out.

Compound A injection has extremely poor solubility in aqueous solvents and is formulated with non-aqueous solvents. It requires dilution with other compatible adjuvants before clinical use. Therefore, during the formulation development stage, the compatibility stability study was conducted simultaneously. The appearance and osmotic pressure of the diluted solutions were examined to preliminarily confirm the appropriate formulation.

### 2.1 Study on Different Surfactants, Formulation Compositions, and Stability

From the surfactant ELP used in the aforementioned experiments, it can be seen that surfactants with certain properties can enhance the solubility of Compound A in absolute ethanol. In the following, formulation compositions with different surfactants and their stability were investigated. The experimental process and results are shown in Table 5.

**Table 5: Investigation of different surfactants, formulation compositions and stability results**

| **Formulations** | **Formulation compositions** | **Operation and experimental observations** | **Investigation conditions** | **Purity (area normalization method)** |
|---|---|---|---|---|
| F5 | 10 mg API, 0.25 g ELP, 0.75 ml absolute ethanol | After the API was added to ELP and absolute ethanol and stirred, it was completely dissolved, and the solution was clear | 0 day | 98.38% |
| | | | -20°C±5°C, 15 days | 98.44% |
| | | | -20°C±5°C, 30 days | 98.06% |
| | | | 5°C±3°C, 15 days | 98.18% |
| | | | 5°C±3°C, 30 days | 97.88% |
| | | | 25°C±5°C, 15 days | 97.96% |
| | | | 25°C±5°C, 30 days | 96.52% |
| F6 | 10 mg API, 0.2 g HS15, 0.8 ml absolute ethanol | After the API was added to HS15 and absolute ethanol and stirred, it was completely dissolved, and the solution was clear | 0 day | 98.26% |
| | | | -20°C±5°C, 15 days | 98.63% |
| | | | -20°C±5°C, 30 days | 98.25% |
| | | | 5°C±3°C, 15 days | 97.91% |
| | | | 5°C±3°C, 30 days | 97.42% |
| | | | 25°C±5°C, 15 days | 95.59% |
| | | | 25°C±5°C, 30 days | 95.45% |
| F7 | 10 mg API, 0.5 ml PEG400, 0.5 ml absolute ethanol | After the API was added to PEG400 and absolute ethanol and stirred, it was completely dissolved, and the solution was clear | 0 day | 98.40% |
| | | | -20°C±5°C, 15 days | 98.43% |
| | | | -20°C±5°C, 30 days | 98.20% |
| | | | 5°C±3°C, 15 days | 98.32% |
| | | | 5°C±3°C, 30 days | 97.95% |
| | | | 25°C±5°C, 15 days | 97.68% |
| | | | 25°C±5°C, 30 days | 97.03% |
| F8 | 10 mg API, 0.5 ml Tween 80, 0.5ml absolute ethanol | After the API was added to Tween 80 and absolute ethanol and stirred, it was completely dissolved, and the solution was clear | 25°C±5°C, 1 days | 98.21% |
| | | | 25°C±5°C, 2 days | 97.93% |
| | | | 25°C±5°C, 3 days | 97.54% |
| | | | 25°C±5°C, 7 days | 96.19% |
| | | | 25°C±5°C, 14 days | 95.33% |
| | | | 25°C±5°C, 21 days | 92.64% |
| | | | 25°C±5°C, 30 days | 89.20% |

| | | | | |
|---|---|---|---|---|
| Note: HS15: relative density (General Rule 0601 of Volume IV of Chinese Pharmacopoeia (2015 Edition)) is 1.06-1.09; ELP: relative density (General Rule 0601 of Volume IV of Chinese Pharmacopoeia (2015 Edition)) is 1.05-1.06. | | | | |

From the above data, it can be seen that Formulation F5 is essentially the same as Formulation F1, wherein the API was completely dissolved in both solvent systems, the solutions were clear, and the purity of the API remained stable under different temperature investigation conditions. The use of different surfactants (Tween 80, HS15, ELP and PEG400) can also increase the solubility of the API in absolute ethanol.

### 2.2 Formulation Design and Preliminary Compatibility Stability Study

According to the different formulation compositions listed in Table 6 below, different concentrated injections of Compound A were prepared, and then were prepared with different compatibility media (0.9% sodium chloride injection, water for injection, and 5% glucose injection) into different compatibility drug solutions. These compatibility solutions were tested for their appearance, osmotic pressure, and pH. The results of the compatibility stability study are shown in Table 7.

**Table 6: Different formulation compositions of concentrated injections of Compound A**

| Formulations | Formulation composition | | | |
|---|---|---|---|---|
| | Compound A (mg) | DMA (g) | ELP (g) | Ethanol |
| F9 | 8 | 0.2 | 0.2 | Added to 2 ml/vial |
| F10 | 40 | 0.2 | 0.2 | Added to 2 ml/vial |
| F11 | 40 | 0.2 | 1 | Added to 2 ml/vial |
| F12 | 40 | 0.2 | 0.8 | Added to 2 ml/vial |
| F13 | 40 | 0.2 | 0.6 | Added to 2 ml/vial |
| F14 | 20 | 0.2 | 1 | Added to 2 ml/vial |
| F15 | 20 | Not added | 0.8 | Added to 2 ml/vial |
| F16 | 20 | Not added | 0.6 | Added to 2 ml/vial |
| F17 | 20 | 0.2 | 0.8 | Added to 2 ml/vial |
| F18 | 20 | Not added | 1 | Added to 2 ml/vial |

**Table 7: Compatibility stability investigation results of Compound A injection**

| Formulations | Compatibility media | Concentration of compatibility solutions | Appearance of compatibility solutions | | Osmotic pressure of compatibility solutions * (mOsmoL/kg) | pH |
|---|---|---|---|---|---|---|
| F9 | 0.9% sodium chloride injection | 0.4 mg/ml | Visually clear but opalescent under light | | 1646.17 | NA |
| | | 0.04 mg/ml | Visually clear but opalescent under light | | 393.48 | NA |
| F10 | 0.9% sodium chloride injection | 0.4 mg/ml | Turbid | | NA | NA |
| | | 0.04 mg/ml | Turbid | | NA | NA |
| F11 | 0.9% sodium chloride injection | 1 mg/ml | Turbid | | 759.41 | NA |
| | water for injection | | Turbid | | 406.36 | NA |
| | 0.9% sodium chloride injection | 0.04 mg/ml | Opalescent | | 330.76 | NA |
| | water for injection | | Turbid | | 0 | NA |
| F12 | 0.9% sodium chloride injection | 1 mg/ml | Extremely turbid | | 837.53 | NA |
| | water for injection | | Turbid | | 436.04 | NA |
| | 0.9% sodium chloride injection | 0.04 mg/ml | Turbid | | 305.22 | NA |
| | water for injection | | Turbid | | 0 | NA |
| F13 | 0.9% sodium chloride injection | 1 mg/ml | Extremely turbid | | 900.93 | NA |
| | water for injection | | Turbid | | 576.52 | NA |
| | 0.9% sodium chloride injection | 0.04 mg/ml | Turbid | | 301.96 | NA |
| | water for injection | | Turbid | | 0 | NA |
| F14 | 0.9% sodium chloride injection | 1 mg/ml | 0h | Opalescent | 1280.33 | NA |
| | | | 4h | Opalescent | | |
| | water for injection | | 0h | Opalescent | 957.35 | NA |
| | | | 4h | Opalescent | | |
| | 5% glucose injection | | 0h | Opalescent | 1323.43 | NA |
| | | | 4h | Opalescent | | |
| | 0.9% sodium chloride injection | 0.04 mg/ml | 0h | Clear | 315.85 | NA |
| | | | 4h | Clear | | |
| | water for injection | | 0h | Clear | 0 | NA |
| | | | 4h | Clear | | |
| | 5% glucose injection | | 0h | Clear | 288.47 | NA |
| | | | 4h | Clear | | |
| F15 | 0.9% sodium chloride injection | 1 mg/ml | 0h | Opalescent | 1494.06 | NA |
| | | | 4h | Opalescent | NA | NA |
| | water for injection | | 0h | Opalescent | 1138.24 | NA |
| | | | 4h | Opalescent | NA | NA |
| | 5% glucose injection | | 0h | Opalescent | 1530.87 | NA |
| | | | 4h | Opalescent | NA | NA |
| | 0.9% sodium chloride injection | 0.04 mg/ml | 0h | Clear | 321.71 | NA |
| | | | 4h | Clear | NA | NA |
| | water for injection | | 0h | Clear | 0 | NA |
| | | | 4h | Clear | NA | NA |
| | 5% glucose injection | | 0h | Clear | 301.07 | NA |
| | | | 4h | Clear | NA | NA |
| F16 | 0.9% sodium chloride injection | 1 mg/ml | 0h | Opalescent | 1725.24 | NA |
| | | | 4h | Opalescent | NA | NA |
| | water for injection | | 0h | Opalescent | 1347.10 | NA |
| | | | 4h | Opalescent | NA | NA |
| | 5% glucose injection | | 0h | Opalescent | 1724.62 | NA |
| | | | 4h | Opalescent | NA | NA |
| | 0.9% sodium chloride injection | 0.04 mg/ml | 0h | Clear | 331.13 | NA |
| | | | 4h | Clear | NA | NA |
| | water for injection | | 0h | Clear | 0 | NA |
| | | | 4h | Clear | NA | NA |
| | 5% glucose injection | | 0h | Clear | 311.93 | NA |
| | | | 4h | Clear | NA | NA |
| F17 | 0.9% sodium chloride injection | 1 mg/ml | 0h | Opalescent | 1442.14 | NA |
| | | | 4h | Opalescent | NA | NA |
| | water for injection | | 0h | Opalescent | 1102.50 | NA |
| | | | 4h | Opalescent | NA | NA |
| | 5% glucose injection | | 0h | Opalescent | 1497.54 | NA |
| | | | 4h | Opalescent | NA | NA |
| | 0.9% sodium chloride injection | 0.04 mg/ml | 0h | Clear | 322.58 | NA |
| | | | 4h | Clear | NA | NA |
| | water for injection | | 0h | Clear | 0 | NA |
| | | | 4h | Clear | NA | NA |
| | 5% glucose injection | | 0h | Clear | 301.12 | NA |
| | | | 4h | Clear | NA | NA |
| F18 | 0.9% sodium chloride injection | 1 mg/ml | 0h | Opalescent | 1335.33 | NA |
| | | | 4h | Clear | NA | NA |
| | water for injection | | 0h | Opalescent | 974.91 | NA |
| | | | 4h | Clear | NA | NA |
| | 5% glucose injection | | 0h | Opalescent | 1365.28 | NA |
| | | | 4h | Clear | NA | NA |
| | 0.9% sodium chloride injection | 0.04 mg/ml | 0h | Opalescent | 309.11 | 5.95 |
| | | | 4h | Clear | NA | 5.86 |
| | water for injection | | 0h | Opalescent | 0 | 5.97 |
| | | | 4h | Clear | NA | 5.95 |
| | 5% glucose injection | | 0h | Opalescent | 285.19 | 4.63 |
| | | | 4h | Clear | NA | 4.75 |
| | | 0.04 mg/ml | 0h | Clear | 270.87/260 | 7.50 |
| | | | 4h | Turbid | NA | 7.52 |

From the preliminary compatibility study results presented in the above table, the following conclusions can be drawn:
① A decrease in the proportion of ELP resulted in an increase in osmotic pressure.
② Nearly all the compatibility solutions of the 20 mg/mL samples appeared turbid, whereas most of the compatibility solutions of the 10 mg/mL samples appeared clear.
③ Form the data for Formulations F14, F15, F16, F17, and F18, it can be seen that the compatibility solutions of these formulations showed no significant differences in appearance or osmotic pressure.
④ Dilution and dissolution of Formulations F10 to F13 with 5% glucose injection as compatibility medium led to clear compatibility solutions.

### 2.3 Screening and Optimization of Formulations

After comprehensively studying the results of compatibility studies of injection formulations with different adjuvants, and the solubility study results of Compound A, and further with consideration of the safety of DMA for injection formulations, the prevalence of adjuvants in injection formulations, and the need to reduce preparation time during manufacturing, five groups of concentrated injection formulations listed in Table 8 were selected for further investigation. The investigation mainly focused on related substances, content, and moisture under different conditions (including different temperatures, storage durations, and light exposure conditions). The results are shown in Table 9. Meanwhile, the compatibility stability, related substances, and content of Formulation F19 were investigated, with the results presented in Table 10, Table 11, and Table 12, respectively.

Furthermore, during the formulation research process, docetaxel injection and paclitaxel injection are often selected as references for comparison of the osmotic pressure of clinically used compatibility dilutions, and serve as references for the clinical use of such products. The comparative data are shown in Table 13.

**Table 8: Different formulation compositions of concentrated injections of Compound A**

| Formulations | Formulation composition | | | |
|---|---|---|---|---|
| | Compound A (mg) | DMA (g) | Compound A (mg) | EtOH |
| F19 | 20 | 0.1 | 0.8 | Added to 2 ml/vial |
| F20 | 20 | 0.1 | 1 | Added to 2 ml/vial |
| F21 | 20 | Not added | 1 | Added to 2 ml/vial |
| F22 | 20 | Not added | 0.8 | Added to 2 ml/vial |
| F23 | 20 | 0.2 | 1 | Added to 2 ml/vial |

The stability data of different concentrated injection formulations of Compound A in Table 8 were investigated. The investigation was conducted under the following conditions: exposure to light or not, different temperatures (25 ± 2°C, 5 ± 3°C, -20 ± 5°C), and different storage durations (0 day, 15 days, 1 month, 2 months, 3 months). The investigation items are as follows and recorded in Table 9.

**Table 9: Stability test results of different concentrated injection formulations of Compound A-related substances, content and moisture**

| **Batch No.** | **Storage conditions** | **Storage duration** | **Related substances** | **Content (by weight)/%** | **Moisture/%** |
|---|---|---|---|---|---|
| | | | **Total impurities** | | |
| F19 | NA | 0 day | 1.96 | 103.8 | 0.5 |
| | 25 ± 2°C | 15 days | 3.24 | 102.1 | 0.4 |
| | | 1 month | 4.61 | 98.9 | 0.5 |
| | | 2 months | 7.48 | 94.4 | 0.4 |
| | | 3 months | 10.42 | 91.9 | 0.4 |
| | 5 ± 3°C | 15 days | 2.13 | 104.1 | 0.4 |
| | | 1 months | 2.22 | 102.3 | 0.4 |
| | | 2 months | 2.35 | 101.2 | 0.4 |
| | | 3 months | 2.76 | 102.8 | 0.4 |
| | -20 ± 5°C | 3 months | 1.96 | 104.7 | 0.5 |
| F20 | NA | 0 day | 1.95 | 102.1 | 0.4 |
| | 25±2°C | 15 days | 3.28 | 99.6 | 0.5 |
| | | 1 month | 4.47 | 97.7 | 0.7 |
| | 5 ± 3°C | 15 days | 2.18 | 100.9 | 0.5 |
| | | 1 month | 2.22 | 101.4 | 0.5 |
| F21 | NA | 0 day | 1.95 | 101.1 | 0.5 |
| | 25 ±2°C | 15 days | 3.38 | 99.7 | 0.5 |
| | | 1 month | 4.74 | 97.8 | 0.5 |
| | 5 ± 3°C | 15 days | 2.11 | 102.3 | 0.5 |
| | | 1 month | 2.24 | 101.2 | 0.5 |
| F22 | NA | 0 day | 1.95 | 102.0 | 0.5 |
| | 25±2°C | 15 days | 3.33 | 101.1 | 0.5 |
| | | 1 month | 4.47 | 98.8 | 0.4 |
| | 5 ± 3°C | 15 days | 2.17 | 102.6 | 0.4 |
| | | 1 month | 2.21 | 102.4 | 0.4 |
| F23 | NA | 0 day | 1.93 | 101.1 | 0.6 |
| | 25 ±2°C | 15 days | 3.03 | 101.4 | 0.6 |
| | | 1 month | 4.29 | 99.1 | 0.6 |
| | | 2 months | 7.67 | 93.6 | 0.6 |
| | | 3 months | 8.94 | 89.8 | 0.8 |
| | 5 ± 3°C | 15 days | 1.98 | 101.8 | 0.6 |
| | | 1 month | 2.04 | 101.9 | 0.6 |
| | | 2 months | 2.47 | 102.8 | 0.6 |
| | | 3 months | 2.54 | 99.6 | 0.6 |
| | -20 ±5°C | 3 months | 1.92 | 100.2 | 0.5 |

The injection formulation F19 (2ml: 20 mg) was prepared with different compatibility media (water for injection, 0.9% sodium chloride injection, 5% glucose injection) into compatibility drug solutions with concentrations of 0.04 mg/ml and 1 mg/ml respectively. Samples were taken at different time points (0 h, 4 h, 6 h, 8 h) to determine the appearance, pH value, osmotic pressure, related substances, content (before and after filtration), etc. The results are shown in Table 10, Table 11 and Table 12, respectively.

The test items, the corresponding test methods and filtration methods of the present invention are as follows:
(1) Appearance: visual inspection;
(2) pH value: 1 ml of the sample was taken and diluted with 0.9% sodium chloride solution to 20 ml, and the pH was measured according to the first method of General Rule 0631 of Volume IV of Chinese Pharmacopoeia (2020 Edition);
(3) Osmotic pressure: dew point osmometer;
(4) Detection of related substances and contents: the detection was conducted using the HPLC method, with detection conditions and parameters similar to those for AST-3424 (please refer to the relevant parts in Patent Publication WO2021/008520, which correspond to paragraphs 133-138 in CN112469394B), wherein the related substances were quantified by the area normalization method, and the content was quantified by the external standard method.

**Table 10: Compatibility stability test results of Formulation F19-appearance, pH, osmotic pressure**

| **Compatibility media** | **Concentration of compatibility solution** | **Time (h)** | **Appearance** | **pH** | **Dew point osmotic pressure (mOsmoL/L)** |
|---|---|---|---|---|---|
| Water for injection | 1 mg/ml | 0 | Opalescent | 5.89 | 40 |
| | | 4 | Opalescent | 6.14 | NA |
| | | 6 | Opalescent | 6.15 | NA |
| | 0.04 mg/ml | 0 | Clear | 5.75 | 0 |
| | | 4 | Clear | 5.94 | NA |
| | | 6 | Clear | 6.07 | NA |
| 0.9% sodium chloride injection | 1 mg/ml | 0 | Opalescent | 5.82 | 306 |
| | | 4 | Opalescent | 5.98 | NA |
| | | 6 | Opalescent | 5.76 | NA |
| | | 8 | Opalescent | 5.81 | NA |
| | 0.04 mg/ml | 0 | Clear | 6.00 | 269 |
| | | 4 | Clear | 6.11 | NA |
| | | 6 | Clear | 6.16 | NA |
| | | 8 | Clear | 5.92 | NA |
| 5% glucose injection | 1 mg/ml | 0 | Opalescent | 5.15 | 293 |
| | | 4 | Opalescent | 5.19 | NA |
| | | 6 | Opalescent | NA | NA |
| | | 8 | Opalescent | 5.31 | NA |
| | 0.04 mg/ml | 0 | Clear | 4.70 | 255 |
| | | 4 | Clear | 4.75 | NA |
| | | 6 | Clear | NA | NA |
| | | 8 | Clear | 4.57 | NA |

**Table 11: Compatibility stability test results of Formulation F 19-related substances**

| **Compatibility media and concentration** | **Storage time** | **Related substances (%)** |
|---|---|---|
| | | **Total impurities** |
| Water for injection-1 mg/ml | 0h | 2.03 |
| | 4h | 2.23 |
| | 6h | 2.53 |
| | 8h | 3.09 |
| Water for injection-0.04 mg/ml | 0h | 2.24 |
| | 4h | 2.96 |
| | 6h | 3.05 |
| 5% glucose injection-1 mg/ml | 0h | 2.2 |
| | 4h | 3.72 |
| | 6h | 4.75 |
| | 8h | 5.59 |
| 5% glucose injection-0.04 mg/ml | 0h | 2.04 |
| | 4h | 5.25 |
| | 6h | 6.68 |
| | 8h | 8.31 |
| 0.9% sodium chloride injection-1 mg/ml | 0h | 2.06 |
| | 4h | 3.19 |
| | 6h | 3.69 |
| | 8h | 4.06 |
| 0.9% sodium chloride injection-0.04 mg/ml | 0h | 1.98 |
| | 4h | 2.42 |
| | 6h | 2.54 |
| | 8h | 2.79 |

**Table 12: Compatibility stability test results of Formulation F19-content (%)**

| **Compatibility media and concentration** | **Before filtration (0h)** | **After filtration (0h)** | **Before filtration (4h)** | **After filtration (4h)** | **Before filtration (6h)** | **After filtration (6h)** | **Before filtration (8h)** | **After filtration (8h)** |
|---|---|---|---|---|---|---|---|---|
| Water for injection-1 mg/ml | 93.7 | 94.3 | 93.5 | 93.1 | 93.3 | 93.9 | 93.1 | 91.3 |
| Water for injection-0.04 mg/ml | 97.4 | 94.9 | 97 | 94.2 | 94.7 | 93 | NA | NA |
| 5% glucose injection-1 mg/ml | 96.9 | 97.2 | 95.7 | 95.1 | 94.3 | 93.9 | 93.4 | 92.2 |
| 5% glucose injection-0.04 mg/ml | 101.6 | 101.5 | 98.2 | 98 | 96.1 | 96.1 | 94.7 | 94.7 |
| 0.9% sodium chloride injection-1 mg/ml | 99.3 | 99.6 | 97.4 | 96 | 96.4 | 96.2 | 95.7 | 93.3 |
| 0.9% sodium chloride injection-0.04 mg/ml | 97.8 | 97.7 | 97.8 | 98.2 | 98.3 | 98.1 | 97.8 | 98.1 |

**Table 13: Osmotic pressure of compatibility solutions of docetaxel injection and paclitaxel injection**

| **Osmotic pressure data of compatibility solutions of paclitaxel injection and docetaxel injection at clinically used concentrations** | | | |
|---|---|---|---|
| **Groups** | **Compatibility media** | **Concentration of compatibility solutions** | **Dew point osmotic pressure (mOsmoL/kg)** |
| Paclitaxel Injection | 0.9% sodium chloride injection | 1.2 mg/ml | 301 |
| | | 0.3 mg/ml | 282 |
| | 5% glucose injection | 1.2 mg/ml | 291 |
| | | 0.3 mg/ml | 268 |
| Docetaxel Injection | 0.9% sodium chloride injection | 0.08 mg/ml | 279 |
| | | 0.74 mg/ml | 267 |

Based on the above data for formulation optimization, the following conclusions can be drawn:
① According to the stability data of different formulations in Table 9, the content and related substances of the five formulations F19-F23 showed similar change trends. The samples were relatively stable when stored at -20±5°C.
② The osmotic pressure of the compatibility solutions prepared from Formulation F19 with 0.9% sodium chloride injection or 5% glucose injection was close to that of compatibility solutions prepared from commercially available docetaxel injection and paclitaxel injection. The osmotic pressure of the compatibility solutions with water for injection was relatively low; therefore, it was not investigated as a compatibility medium in subsequent studies.
③ The solutions formulated with 0.9% sodium chloride injection or 5% glucose injection exhibited opalescence. The content determination of the samples before and after filtration revealed that the content of the solutions formulated with 0.9% sodium chloride injection or 5% glucose injection fluctuated by less than 2% before and after filtration. This indicates that the opalescence of the compatibility solutions prepared from Compound A with 0.9% sodium chloride injection or 5% glucose injection was not due to incomplete dissolution of the product and does not affect their use.

### 2.4 Compatibility Stability Study

Based on the confirmed Formulation F19, an injection was prepared using raw and auxiliary materials of the same quality as those used in GMP production, and a compatibility stability study was conducted. The preliminary compatibility stability results indicated that the related substances in the compatibility solutions increased rapidly at room temperature. Considering the physicochemical properties of the active pharmaceutical ingredient, the pH of the compatibility solvents was investigated. 5% sodium bicarbonate injection was first used to adjust the pH values of 5% glucose injection and 0.9% sodium chloride injection to 7.0 and 8.0, respectively, and then the aforementioned solutions were used for the compatibility stability study.

The compatibility stability study results are show in Table 14.

**Table 14: Compatibility stability results -appearance, pH, osmotic pressure, related substances**

| **Compatibility media** | **Concentration of compatibility solutions** | **Time (h)** | **Appearance** | **pH of compatibility solutions** | **Dew point osmotic pressure (mOsm/kg** ) | **Related substances (total impurities/%)** |
|---|---|---|---|---|---|---|
| 0.9% sodium chloride injection (pH 7.0) | 1 mg/ml | 0 | Opalescent | 6.55 | 317 | 0.6 |
| | | 4 | Opalescent | 6.81 | NA | 1.4 |
| | | 8 | Opalescent | 6.7 | NA | 1.4 |
| | 0.08 mg/ml | 0 | A clear solution | 6.75 | 293 | 0.6 |
| | | 4 | A clear solution | 6.74 | NA | 1.4 |
| | | 8 | A clear solution | 6.68 | NA | 1.4 |
| 0.9% sodium chloride injection (pH 8.0) | 1 mg/ml | 0 | Opalescent | 7.1 | 320 | 0.6 |
| | | 4 | Opalescent | 7.01 | NA | 0.7 |
| | | 8 | Opalescent | 7.33 | NA | 0.8 |
| | 0.08 mg/ml | 0 | A clear solution | 7.41 | 294 | 0.6 |
| | | 4 | A clear solution | 7.38 | NA | 0.7 |
| | | 8 | A clear solution | 7.37 | NA | 0.8 |
| 5% glucose injection (pH 7.0) | 1 mg/ml | 0 | Opalescent | 7.26 | 293 | 0.6 |
| | | 4 | Opalescent | 7.07 | NA | 0.6 |
| | | 8 | Opalescent | 7.22 | NA | 0.7 |
| | 0.08 mg/ml | 0 | A clear solution | 7.32 | 261 | 0.6 |
| | | 4 | A clear solution | 7.1 | NA | 0.6 |
| | | 8 | A clear solution | 7.19 | NA | 0.7 |
| 5% glucose injection (pH 8.0) | 1 mg/ml | 0 | Opalescent | 7.46 | 303 | 0.6 |
| | | 4 | Opalescent | 7.66 | NA | 0.6 |
| | | 8 | Opalescent | 7.79 | NA | 0.7 |
| | 0.08 mg/ml | 0 | A clear solution | 7.86 | 266 | 0.6 |
| | | 4 | A clear solution | 8.03 | NA | 1.1 |
| | | 8 | A clear solution | 7.92 | NA | 0.6 |

As can be seen from the data in Table 14, the three groups of compatibility solutions with 0.9% sodium chloride injection (pH 8.0), 5% glucose injection (pH 7.0), and 5% glucose injection (pH 8.0) respectively have a pH greater than 7.0. After being placed at room temperature for 8 hours, the related substances in these solutions remained relatively stable, and the pH and osmotic pressure of the compatibility solutions met the requirements for injection. The compatibility stability of Formulation F19 (20 mg Compound A + 0.1 g DMA + 0.8 g ELP + ethanol added to 2 ml) met the requirements for injection.

### III. Influence Factor Study of Compound A Injection

### 3.1 Influence Factor Tests

The Compound A injection (2 mL: 20 mg, Formulation F19) was prepared and stored and treated under the experimental conditions listed in Table 15 below. Tests were carried out according to the sampling time points and test items specified in Table 16 below. The results for all test items under high temperature (25°C ± 2°C) storage, light exposure (without outer packaging), and light exposure (with outer packaging) were recorded in Tables 17, 18, and 19, wherein T₀ data represent the results of simultaneous testing of the samples stored at -20°C at day 5.

**Table 15: Conditions for the influence factor tests**

| Conditions | Parameters |
|---|---|
| High temperature | 25°C±2 °C |
| Light exposure (without outer packaging, 15°C) | 4500 Lux±500 Lux, total illumination not less than 1.2×10⁶ Lux·hr; near ultraviolet energy not less than 200 w·hr/m² |
| Light exposure (with outer packaging, 15°C) | 4500 Lux±500 Lux, total illumination of not less than 1.2×10⁶ Lux·hr; near ultraviolet energy not less than 200 w·hr/m² |

**Table 16: Sampling time points and test items for the influence factor tests**

| Storage conditions | Sampling time | | | |
|---|---|---|---|---|
| | T₀ | Day 5 | Day 10 | Day 30 |
| -20°C±5°C | A | - | - | - |
| High temperature (25°C±2 °C) | - | A | A | A |
| Light exposure (without outer packaging, 15°C) | - | A | A | A |
| Light exposure (with outer packaging, 15°C) | - | A | A | A |

| | | | | |
|---|---|---|---|---|
| A=Trait, moisture, pH value, visible foreign matter, content, and related substances. | | | | |

**Table 17: Test results of the influence factor of high temperature (25°C ± 2°C) storage**

| **Storage condition: 25°C±2°C** | | | | | | | |
|---|---|---|---|---|---|---|---|
| **Test items** | **Analytical method** | **Acceptance criteria** | | **T₀** | **Day 5** | **Day 10** | **Day 30** |
| Trait | Visual inspection | Light-yellow clear solution | | Light-yellow clear solution | Light-yellow clear solution | Light-yellow clear solution | Light-yellow clear solution |
| pH | General Rule 0631 of Chinese Pharmacopoeia (2020 Edition) | 4.5-7.0 | | 5.9 | 5.9 | 6 | 6.1 |
| Visible foreign matter | First method of General Rule 0904 of Chinese Pharmacopoeia (2020 Edition) | Meeting the regulations | | Meeting the regulations | Meeting the regulations | Meeting the regulations | Meeting the regulations |
| Related substances | HPLC area normalization method | Total impurities | ≤5.0% | 0.50% | 0.90% | 1.60% | 2.90% |
| Moisture | First method of General Rule 0832 of Chinese Pharmacopoeia (2020 Edition) | ≤1.0% | | 0.20% | 0.30% | 0.20% | 0.20% |
| Content | HPLC external standard method | 90%-110% (nominal amount) | 100.10% | 100.40% | 98.50% | 96.70% | |

**Table 18: Test results of the influence factor of light exposure (without outer packaging)**

| **Storage conditions: light exposure (without outer packaging)** | | | | | | | |
|---|---|---|---|---|---|---|---|
| **Test items** | **Analytical method** | **Acceptance criteria** | | **Time points** | | | |
| | | | | **T₀** | **Day 5** | **Day 10** | **Day 30** |
| Trait | Visual inspection | Light-yellow clear solution | | Light-yellow clear solution | Light-yellow clear solution | Light-yellow clear solution | Light-yellow clear solution |
| pH | General Rule 0631 of Chinese Pharmacopoeia (2020 Edition) | 4.5-7.0 | | 5.9 | 5.9 | 6 | 6.1 |
| Visible foreign matter | First method of General Rule 0904 of Chinese Pharmacopoeia (2020 Edition) | Meeting the regulations | | Meeting the regulations | Meeting the regulations | Meeting the regulations | Meeting the regulations |
| Related substances | HPLC area normalization method | Total impurities | ≤5.0 % | 0.50% | 0.70% | 0.90% | 1.30% |
| Moisture | First method of General Rule 0832 of Chinese Pharmacopoeia (2020 Edition) | ≤1.0% | | 0.20% | 0.20% | 0.20% | 0.20% |
| Content | HPLC external standard method | 90%-110% (nominal amount) | 100.10% | 99.50% | 99.70% | 99.00% | |

**Table 19: Test results of the influence factor of light exposure (with outer packaging)**

| **Storage conditions: light exposure (with outer packaging)** | | | | | | | |
|---|---|---|---|---|---|---|---|
| **Test items** | **Analytical method** | **Acceptance criteria** | | **Time points** | | | |
| | | | | **T₀** | **Day 5** | **Day 10** | **Day 30** |
| Traits | Visual inspection | Light-yellow clear solution | | Light-yellow clear solution | Light-yellow clear solution | Light-yellow clear solution | Light-yellow clear solution |
| pH | General Rule 0631 of Chinese Pharmacopoeia (2020 Edition) | 4.5-7.0 | | 5.9 | 5.9 | 6 | 6.1 |
| Visible foreign matter | First method of General Rule 0904 of Chinese Pharmacopoeia (2020 Edition) | Meeting the regulations | | Meeting the regulations | Meeting the regulations | Meeting the regulations | Meeting the regulations |
| Related substances | HPLC area normalization method | Total impurities | ≤5.0% | 0.50% | 0.70% | 0.90% | 1.30% |
| Moisture | First method of General Rule 0832 of Chinese Pharmacopoeia (2020 Edition) | ≤1.0% | | 0.20% | 0.20% | 0.20% | 0.20% |
| Content | HPLC external standard method | 90%-110% (nominal amount) | 100.10% | 100.20% | 98.10% | 99.10% | |

The data in Tables 17, 18, and 19 above indicate that:
① Appearance: After 30 days of storage under both high temperature (25°C±2°C) conditions and light exposure conditions, the appearance of Injection Formulation F19 remained as a light-yellow clear liquid with no significant changes.
② pH: After 30 days of storage under both high temperature (25°C±2°C) conditions and light exposure conditions, the pH of Injection Formulation F19 remained within the range of 4.5 to 7.0, with no significant changes.
③ Visible foreign matter: After 30 days of storage under both high temperature (25°C±2°C) conditions and light exposure conditions, the visible foreign matter in Injection Formulation F19 met the standard regulations.
④ Total impurities: After 30 days of storage under high temperature (25°C±2°C) conditions, the percentage of the total impurities of Injection Formulation F19 was lower than the standard limit of 5.0%. After 30 days of storage under both light exposure conditions (with outer packaging, at the illuminance specified in the Chinese Pharmacopoeia, 15°C) and light exposure conditions (without outer packaging, at the illuminance specified in the Chinese Pharmacopoeia, 15°C), the percentage of the total impurities was lower than the standard limit of 5.0%. The increase in impurities under light exposure conditions (with outer packaging) was identical to that observed under light exposure conditions (without outer packaging), and the amount of increase under both light exposure conditions was lower than that observed under high temperature (25°C±2°C) conditions. All results met the quality standard regulations.
⑤ Moisture: After 30 days of storage under both high temperature (25°C±2°C) conditions and light exposure conditions, the moisture content of Injection Formulation F19 was no more than 0.3%, meeting the regulations.
⑥ Content: After 30 days of storage under high temperature (25°C±2°C) conditions, the content of Injection Formulation F19 decreased by 3.4%, meeting the quality standard regulations; after 30 days of storage under both light exposure conditions (with outer packaging, at the illuminance specified in the Chinese Pharmacopoeia, 15°C) and light exposure conditions (without outer packaging, at the illuminance specified in the Chinese Pharmacopoeia, 15°C), the content of Injection Formulation F19 showed no significant fluctuations and met the quality standard requirements.

### 3.2 Freeze-Thaw Test

The Compound A injection (2 mL: 20 mg, Formulation F19) was prepared and subjected to freeze-thaw treatment under the test conditions listed in Table 20 below. Samples were taken for freeze-thaw test according to the sampling time points and test items listed in Table 21 below. The test results for all test items under the two freeze-thaw test conditions are presented in Tables 22 and 23, respectively, wherein the initial data from the influence factor tests described in Section 3.1 were used as the T₀ data.

### Experimental Procedures:

Freeze-Thaw Cycle 1: The prepared Compound A injection (2 mL: 20 mg) was stored at -20°C for 2 days, and then transferred to an environment of 2-8°C for storage for 2 days, which constituted a temperature cycle. Such temperature cycle was conducted for three times, and the samples were analyzed after each temperature cycle. The initially prepared Compound A injection was stored in a -20°C freezer as a control.

Freeze-Thaw Cycle 2: The above process was repeated for a temperature cycle from -20°C to 25°C.

**Table 20: Freeze-thaw test conditions**

| Conditions | Parameters |
|---|---|
| Freeze-thaw 1 | -20°C±5 °C (2 days)→5°C±3 °C (2 days) |
| Freeze-thaw 2 | -20°C±5 °C (2 days)→25°C±2 °C (2 days) |

**Table 21: Sampling time points and test items for freeze-thaw test**

| Storage conditions | Sampling time points | | | |
|---|---|---|---|---|
| | T₀ | First cycle | Second cycle | Third cycle |
| -20°C±5°C | A | - | - | - |
| Freeze-thaw 1 | - | A | A | A, M |
| Freeze-thaw 2 | - | A | A | A, M |

| | | | | |
|---|---|---|---|---|
| Note: A = Trait, moisture, pH value, visible foreign matter, content, related substances; M = sterility, bacterial endotoxins. | | | | |

**Table 22: Test results of freeze-thaw cycle 1**

| **Storage conditions for freeze-thaw cycle 1: -20°C±5 °C (2 days)→5°C±3 °C (2 days)** | | | | | | | |
|---|---|---|---|---|---|---|---|
| **Test items** | **Analytical method** | **Acceptance criteria** | | **Time points** | | | |
| | | | | **T₀** | **First cycle** | **Second cycle** | **Third cycle** |
| Trait | Visual inspection | Light-yellow clear solution | | Light-yellow clear solution | Light-yellow clear solution | Light-yellow clear solution | Light-yellow clear solution |
| pH | General Rule 0631 of Chinese Pharmacopoeia (2020 Edition) | 4.5-7.0 | | 5.9 | 6 | 6 | 6 |
| Visible foreign matter | First method of General Rule 0904 of Chinese Pharmacopoeia (2020 Edition) | Meeting the regulations | | Meeting the regulations | Meeting the regulations | Meeting the regulations | Meeting the regulations |
| Related substances | HPLC area normalization method | Total impurities | ≤5.0 % | 0.50% | 0.60% | 0.60% | 0.60% |
| Moisture | First method of General Rule 0832 of Chinese Pharmacopoeia (2020 Edition) | ≤1.0% | | 0.20% | 0.30% | 0.20% | 0.20% |
| Sterility | General Rule 1101 of Chinese Pharmacopoeia (2020 Edition) | No microbial growth should be observed. | NA | NA | NA | Meeting the regulations | |
| Bacterial endotoxins | General Rule 1143 of Chinese Pharmacopoeia (2020 Edition) | <2EU/ mg | NA | NA | NA | <1.2EU/ mg | |
| **Content** | HPLC external standard method | 90%-110% (nominal amount) | 100.10% | 99.70% | 99.50% | 99.60% | |

| | | | | | | | |
|---|---|---|---|---|---|---|---|
| Note: NA means not applicable; the T₀ data from the influence factor tests were used as the initial data. | | | | | | | |

**Table 23: Test results of freeze-thaw cycle 2**

| **Storage conditions for freeze-thaw cycle 2: -20°C±5 °C (2 days)→25°C±2 °C (2 days)** | | | | | | | |
|---|---|---|---|---|---|---|---|
| **Test items** | **Analytical method** | **Acceptance criteria** | | **Time points** | | | |
| | | | | **T₀** | **First cycle** | **Second cycle** | **Third cycle** |
| Trait | Visual inspection | Light-yellow clear solution | | Light-yellow clear solution | Light-yellow clear solution | Light-yellow clear solution | Light-yellow clear solution |
| pH | General Rule 0631 of Chinese Pharmacopoeia (2020 Edition) | 4.5-7.0 | | 5.9 | 5.9 | 6 | 6 |
| Visible foreign matter | First method of General Rule 0904 of Chinese Pharmacopoeia (2020 Edition) | Meeting the regulations | | Meeting the regulations | Meeting the regulations | Meeting the regulations | Meeting the regulations |
| Related substances | HPLC area normalization method | Total impurities | ≤5.0 % | 0.50% | 0.70% | 0.90% | 1.10% |
| Moisture | First method of General Rule 0832 of Chinese Pharmacopoeia (2020 Edition) | ≤1.0% | | 0.20% | 0.20% | 0.20% | 0.20% |
| Sterility | General Rule 1101 of Chinese Pharmacopoeia (2020 Edition) | No microbial growth should be observed. | NA | NA | NA | Meeting the regulations | |
| Bacterial endotoxins | General Rule 1143 of Chinese Pharmacopoeia (2020 Edition) | <2EU/ mg | NA | NA | NA | <1.2EU/ mg | |
| Content | HPLC external standard method | 90%-110% (nominal amount) | 100.10% | 99.70% | 98.70% | 98.60% | |

| | | | | | | | |
|---|---|---|---|---|---|---|---|
| Note: NA means not applicable; the T₀ data from the influence factor tests were used as the initial data. | | | | | | | |

The data in Tables 22 and 23 above indicate:
① Appearance: After three cycles under both freeze-thaw conditions, Injection Formulation F19 showed no significant change in the appearance and remained as a light-yellow clear liquid.
② pH: After three cycles under both freeze-thaw conditions, the pH of Injection Formulation F19 remained within the range of 4.5 to 7.0, with no significant changes.
③ Visible foreign matter: After 3 cycles under both freeze-thaw conditions, the visible foreign matter met the regulations.
④ Total impurities: After 3 freeze-thaw cycles under freeze-thaw 1 conditions (-20°C±5°C for 2 days → 5°C±3°C for 2 days), the total impurities in Injection Formulation F19 remained basically unchanged. After 3 freeze-thaw cycles under freeze-thaw 2 conditions (-20°C±5°C for 2 days → 25°C±2°C for 2 days), new unknown single impurities were detected. In both cases, the level of new impurities was not more than 0.1%, which met the quality standard regulations.
⑤ Moisture: After 3 cycles under both freeze-thaw conditions, the moisture content of Injection Formulation F19 was no more than 0.3%, which met the regulations.
⑥ Sterility: After the 3rd cycle under both freeze-thaw conditions, no bacterial growth was detected in Injection Formulation F19, which met the regulations.
⑦ Bacterial endotoxins: After the 3rd cycle under both freeze-thaw conditions, the bacterial endotoxin level of Injection Formulation F19 was less than 1.2 EU/mg, which met the regulations.
⑧ Content: After three freeze-thaw cycles under both freeze-thaw 1 conditions (-20°C±5°C for 2 days → 5°C±3°C for 2 days) and freeze-thaw cycle 2 conditions (-20°C±5°C for 2 days → 25°C±2°C for 2 days), the content of Injection Formulation F19 showed no significant fluctuations and met the quality standard regulations.

### IV. Preparation Process and Specific Examples of Concentrated Injections of Compound A

The preparation of concentrated injections of Compound A used in the above experiments can all be prepared by the following process:
(1) Cleaning and sterilization of rubber stoppers
   The rubber stoppers were cleaned, sterilized and dried before use (steam sterilization conditions: 121°C, 30 minutes).
(2) Cleaning and sterilization of aluminum caps
   The aluminum caps were cleaned, sterilized and dried before use (steam sterilization conditions: 121°C, 30 minutes).
(3) Cleaning and sterilization of vials
   The vials were cleaned, dry-heat sterilized and cooled before use.
(4) Weighing
   The active ingredient and adjuvants were weighed according to the prescribed amounts.
(5) Preparation of a solution and filtration
   Taking Formulations F9 to F14, F19, F20, F23, etc., as examples:
   the pre-weighed Compound A was added to the pre-weighed N,N-dimethylacetamide (DMA, for injection), and stirred until it was dissolved, and then the mixture was set aside for subsequent use.

A portion of absolute ethanol was added into a liquid preparation tank (temperature controlled at 15-20°C). Then, the pre-weighed polyoxyethylene (35) castor oil (ELP) was added. The container was rinsed with an appropriate amount of absolute ethanol, and stirring was initiated until the solution became visually clear.

The prepared solution of Compound A and N,N-dimethylacetamide (for injection) was slowly poured into the liquid preparation tank. The container was rinsed with an appropriate amount of absolute ethanol, and stirring was continued for ≥ 10 minutes until the solution became visually clear. Additional absolute ethanol was added to reach the total weight of the formulation, and stirring was continued for ≥ 20 minutes.

Samples of the intermediates were taken for testing. The test items included trait, pH value, moisture, density, content, bacterial endotoxins, moisture and microbial load.

Two 0.22 µm sterilizing-grade polytetrafluoroethylene capsule filters were connected in series, and the drug solution was sterilized and filtered to the mobile liquid receiving tank at the filling station using compressed air.

### (6) Filling and rimp-capping

The filling volume was calculated and adjusted using the weighing method. After the filling volume was qualified after adjustment, filling and half stoppering were started. The intermediate products after filling and full stoppering was transported to the capping room for capping.

### 7. Lamp inspection and packaging

Lamp inspection was conducted on the intermediate products after capping. The intermediate products that passed the lamp inspection were labeled, and samples of the finished products were taken for release testing, followed by packaging.

Taking Formulations F9 to F14, F19, F20, F23, etc., as examples, the production process flowchart of the concentrated Compound A (compound of formula I) injection formulations is shown in Figure 1.

The present invention also provides another process for preparing Compound A-containing injections, which is mainly for preparing lab-scale batches of injection formulations. This process differs from the aforementioned process in the solution preparation process, and more particularly, in the sequence of adding ingredients. The specific approach and steps are as follows:
The concentration of the Compound A injection product is 10 mg/ml, and its formulation includes one active ingredient and three adjuvants. Since the solubility of the active ingredient Compound A decreases sequentially in the three adjuvants (DMA, ELP, and absolute ethanol), the active pharmaceutical ingredient (API) Compound A is first added to the prescribed amount of DMA and stirred until it is dissolved. After Compound A is completely dissolved in DMA, the prescribed amount of ELP is added and stirring is continued. Finally, absolute ethanol is added to the final volume so as to reduce the risk of precipitation during the dissolution of the API. During the operation, it was observed that the subsequently added ELP and absolute ethanol dissolved rapidly, and the final solution was clear and transparent.

### V. Compatibility and Stability Study of Ready-to-Use Intravenous Injections

The formulation design, properties such as stability, and specific preparation examples of the concentrated injections have been studied above. The following will describe the preparation process and stability studies of ready-to-use injections.

In order to provide compatibility information for the injection of the compound of formula I (Compound A, API) and the compatibility solutions, it is necessary to investigate the short-term stability of the injection in 5% glucose injection, a commonly used intravenous diluent. Considering that the API is instable in acidic environments, the pH of the 5% glucose injection is first adjusted to 7.4 using a sodium bicarbonate solution.

### 5.1 Experimental Methods and Procedures

### Adjustment of the pH of the compatibility medium:

1.0 ml of 5% sodium bicarbonate injection was added to 250 ml of 5% glucose injection to adjust the pH, and mixed homogeneously. At this point, the pH of the mixed solution was approximately 8.0.

### Preparation of compatibility solutions of Compound A:

The Compound A injection (2 ml: 20 mg) was prepared with the pH-adjusted 5% glucose injection into a compatibility solution with a concentration of 0.02 mg/ml, and a compatibility solution with a concentration of 1.00 mg/ml.

### Preparation of a 0.02 mg/ml compatibility solution:

0.501 ml of Compound A injection (2 ml: 20 mg) was added to a mixing bag containing 250 ml of 5% glucose injection (whose pH had been pre-adjusted using 5% sodium bicarbonate injection). The mixing bag was repeatedly inverted to ensure homogeneous mixing of the mixed solution before storage and sampling.

### Preparation of a 1.00 mg/ml compatibility solution:

27.8 ml of Compound A injection (2 ml: 20 mg) was added to a mixing bag containing 250 ml of 5% glucose injection (whose pH had been pre-adjusted using 5% sodium bicarbonate injection). The mixing bag was repeatedly inverted to ensure homogeneous mixing of the mixed solution before storage and sampling.

### Temperature and light conditions:

All compatibility solution samples were stored under indoor ambient lighting conditions at room temperature (25±3°C).

### Sampling time points and test items:

0h (before and after filtration), 2h, 4h, and 8h. Appearance, color of solution, clarity of solution, pH value, osmotic pressure, related substances, and content.

### 5.2 Experimental Results

All the experimental data are listed in Tables 24 and 25 below, which summarize the stability data of compatibility solutions with different concentrations.

**Table 24: Stability test results of compatibility drug solutions with a concentration of 0.02 mg/ml**

| **Test items** | **0h** | | **2h** | **4h** | **8h** |
|---|---|---|---|---|---|
| | **Before filtration** | **After filtration** | | | |
| Appearance | Colorless clear liquid | Colorless clear liquid | Colorless clear liquid | Colorless clear liquid | Colorless clear liquid |
| Color of solution | Colorless | Colorless | Colorless | Colorless | Colorless |
| Clarity | Clear solution | Clear solution | Clear solution | Clear solution | Clear solution |
| Osmotic pressure | 263 | 265 | NA | NA | 271 |
| pH value | 8.1 | 8.1 | 7.9 | 8.1 | 8.1 |
| Content (mg/ml) | 0.019 | 0.018 | 0.018 | 0.019 | 0.018 |
| Related substances-total impurities | 0.9% | 0.9% | 0.9% | 0.9% | 0.9% |

**Table 25: Stability test results of compatibility drug solutions with a concentration of 1.00 mg/ml**

| **Test items** | **0h** | | **2h** | **4h** | **8h** |
|---|---|---|---|---|---|
| | **Before filtration** | **After filtration** | | | |
| Appearance | Light yellow liquid with opalescence | Light yellow liquid with opalescence | Light yellow liquid with opalescence | Light yellow liquid with opalescence | Light yellow liquid with opalescence |
| Color of solution | Light yellow | Light yellow | Light yellow | Light yellow | Light yellow |
| Clarity | Between turbidity standard solutions No. 2 and No. 3 | Between turbidity standard solutions No. 2 and No. 3 | Between turbidity standard solutions No. 2 and No. 3 | Between turbidity standard solutions No. 2 and No. 3 | Between turbidity standard solutions No. 2 and No. 3 |
| Osmotic pressure | 303 | 309 | NA | NA | 304 |
| pH value | 7.8 | 7.7 | 7.7 | 7.8 | 7.8 |
| Content (mg/ml) | 0.980 | 0.978 | 0.982 | 0.979 | 0.928* |
| Related substances-Total impurities | 1.0% | 1.0% | 0.8% | 0.8% | 0.9% |

| | | | | | |
|---|---|---|---|---|---|
| Note: *: Since the drug content of the 1.0 mg/ml compatibility solutions at 8h decreased by more than 5% compared with that at 0 h, an additional test at 6h was conducted (see Table 26), wherein the injection samples used for preparing the compatibility solutions were from the same batch. | | | | | |

**Table 26: Additional stability test results of compatibility drug solutions at 6h with a concentration of 1.00 mg/ml**

| **Test items** | **Oh*** | | **6h*** |
|---|---|---|---|
| | **Before filtration** | **After filtration** | |
| Appearance | Nearly colorless liquid with opalescence | Nearly colorless liquid with opalescence | Nearly colorless liquid with opalescence |
| Color of solution | Nearly colorless | Nearly colorless | Nearly colorless |
| Clarity | Between turbidity standard solutions No. 1 and No. 2 | Between turbidity standard solutions No. 1 and No. 2 | Between turbidity standard solutions No. 1 and No. 2 |
| Osmotic pressure | 296 | 301 | 290 |
| pH value | 7.7 | 7.8 | 7.9 |
| Content (mg/ml) | 0.948 | 0.950 | 0.950 |
| Related substances-total impurities | 1.0% | 1.1% | 0.8% |

The experimental data in Tables 24, 25 and 26 show that:
The 0.02 mg/ml compatibility solutions showed no changes in traits, clarity, and content before and after filtration through a precision filtration infusion set. After the filtration, the compatibility solutions exhibited no significant changes in traits, color and clarity of solution, osmotic pressure, pH value, content of Compound A, and related substances within 8 hours

The 1.00 mg/ml compatibility solutions showed no changes in traits, clarity, and content before and after filtration through a precision filtration infusion set. After the filtration, the compatibility solutions exhibited no significant changes in traits, color and clarity of solution, osmotic pressure, pH value, and content of Compound A within 6 hours

Before infusion, the injection of Formulation F19 needs to be diluted with pH-adjusted 5% glucose injection (whose pH has been pre-adjusted by adding 1.0 ml of 5% sodium bicarbonate injection into 250 ml of 5% glucose injection). When diluted to a concentration ranging from 0.02 mg/ml to 1 mg/ml, the solution remains stable for 6 hours at room temperature under indoor ambient lighting. The diluted solution may show opalescence, which is caused by the adjuvants in the injection. It has been confirmed that after filtration through a disposable precision filtration infusion set, the content remains unchanged within 6 hours.

### VI. Chiral Stability Study of Compound A in Compound A Injection and in Compatibility Drug Solution of Compound A Injection

Compound A is a racemic mixture containing one chiral center. Its two isomers, named A-P1 and A-P2, can be obtained by resolving Compound A. During the formulation and compatibility studies, interconversion between the chiral isomers may affect the drug safety. Therefore, it is necessary to study the chiral stability of Compound A in both its injection formulation and the compatibility drug solution of the injection formulation.

### Preparation of a solution:

(a) The concentrated injection formulation of Compound A (2 ml: 20 mg) was prepared according to the aforementioned confirmed Formulation F19 and its preparation method.
(b) The compatibility drug solution with a concentration of 1.00 mg/ml was prepared according to the process for preparing a compatibility solution as described in Section 5.1.

### Experimental methods and condition control:

For the injection formulation of Compound A (concentrated injection), the formulation was stored under light-protected, sealed conditions at -20±5°C. Samples were taken at different time points (0 month, 1 month, 3 months), and the contents of A-P1 and A-P2 were determined using the aforementioned HPLC method for stability evaluation. The results are shown in Table 27.

For the 1.00 mg/ml compatibility drug solution (ready-to-use injection), the solution was stored at room temperature (25°C ± 2°C) under indoor ambient lighting. Samples were taken at different time points (0 h, 2 h, 4 h, 8 h and 24 h) and the contents of A-P1 and A-P2 were determined using the aforementioned HPLC method to evaluate chiral stability. The results are shown in Table 28.

**Table 27: Chiral stability study of the concentrated Compound A injection**

| Injection | Isomer peak | Results for isomers (%) | | |
|---|---|---|---|---|
| | | 0 month | 1 month | 3 months |
| | AST-001-P1 | 50.0 | 50.0 | 50.0 |
| Compound A Injection | AST-001-P2 | 50.0 | 50.0 | 50.0 |
| | **Isomer interconversion** | **NA** | **0.0** | **0.0** |

**Table 28: Chiral stability study of the compatibility drug solution of Compound A**

| Solution | Isomer peak | Results for isomers (%) | | | | |
|---|---|---|---|---|---|---|
| | | 0h | 2h | 4h | 8h | 24h |
| Compatibility solution of Compound A injection | AST-001-P1 | 50.0 | 50.0 | 50.0 | 50.0 | 50.0 |
| | AST-001-P2 | 50.0 | 50.0 | 50.0 | 50.0 | 50.0 |
| | **Isomer interconversion** | **NA** | **0.0** | **0.0** | **0.0** | **0.0** |

The experimental data above indicate that Compound A is chirally stable in both its injection formulation and the compatibility solution of the injection formulation, with almost no chiral interconversion between the two isomers.

## Claims

1. An injection preparation, which is a solution containing a compound of the following formula (I), wherein the solvent of the solution contains a C2-C8 alcohol or a liquid mixture thereof:

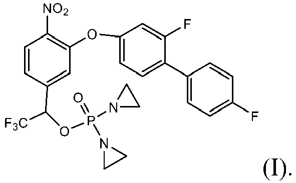

2. The injection preparation according to claim 1, wherein the C2-C8 alcohol is a monohydric alcohol or a polyhydric alcohol, preferably a monohydric alcohol, and more preferably ethanol.

3. The injection preparation according to claim 1, wherein the solvent of the solution further comprises a non-ionic surfactant as a solubilizer.

4. The injection preparation according to claim 3, wherein the non-ionic surfactant is selected from polyethylene glycols, hydroxystearate esters, polyoxyethylene alkyl ethers, Tweens, Spans, or polyoxyethylene castor oils;
the polyethylene glycols include polyethylene glycol 200, polyethylene glycol 400, polyethylene glycol 600, and polyethylene glycol 800;
the hydroxystearate esters include 15-hydroxystearate polyethylene glycol ester and 12-hydroxystearate polyethylene glycol ester;
the alkyl groups in the polyoxyethylene alkyl ethers include lauryl ether alkyl, oleyl ether alkyl, stearyl alcohol alkyl, and hexadecyl;
the Tweens include Tween 20, Tween 40, Tween 60, and Tween 80;
the Spans include Span 20, Span 40, Span 60, and Span 80;
the polyoxyethylene castor oils include polyoxyethylene castor oil, and polyoxyethylene hydrogenated castor oil.

5. The injection preparation according to claim 1, wherein the solvent further comprises one or more of polyoxyethylene (35) castor oil, 15-hydroxystearate polyethylene glycol, polyethylene glycol 400, and Tween 80.

6. The injection preparation according to claim 3, wherein the volume ratio of the non-ionic surfactant to the C2-C8 alcohol in the solvent of the solution is 1:(1-4).

7. The injection preparation according to claim 1, wherein the solvent of the solution further comprises an organic solvent with a hydrotropic effect.

8. The injection preparation according to claim 7, wherein the organic solvent with a hydrotropic effect is selected from ethylenediamine, N,N-dimethylacetamide, N,N-dimethylformamide or polyvinylpyrrolidone, and preferably N,N-dimethylacetamide.

9. The injection preparation according to claim 7, wherein the mass ratio of the organic solvent with a hydrotropic effect in the solvent of the solution to the compound of formula (I) in claim 1 is (10-50):1.

10. The injection preparation according to claim 1, wherein the solvent of the solution further comprises a non-ionic surfactant as a solubilizer and an organic solvent with a hydrotropic effect.

11. The injection preparation according to claim 10, wherein the non-ionic surfactant is selected from polyoxyethylene (35) castor oil, and the organic solvent with a hydrotropic effect is selected from N,N-dimethylacetamide.

12. The injection preparation according to claim 10, wherein the mass ratio of the non-ionic surfactant in the solvent of the solution to the compound of formula (I) in claim 1 is (0-90):1, preferably (5-50):1, more preferably (15-40):1, more preferably (20-40):1, and further more preferably (25-40):1; or
the mass ratio of the organic solvent with a hydrotropic effect in the solvent of the solution to the compound of formula (I) in claim 1 is (0-100):1, preferably (1-50):1, more preferably (5-25):1, and further more preferably (5-10):1.

13. The injection preparation according to any one of claims 1 to 12, wherein the content of the compound of formula (I) is 0.1-200 mg/ml, preferably 1-100 mg/ml, and further preferably 10-20 mg/ml.

14. An injection preparation, comprising the compound of formula (I) according to claim 1, N,N-dimethylacetamide, polyoxyethylene (35) castor oil and ethanol.

15. An injection preparation, which consists of the compound of the following formula (I), N,N-dimethylacetamide, polyoxyethylene (35) castor oil and ethanol:

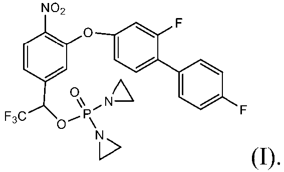

16. The injection preparation according to claim 14 or 15, wherein,
the content of the compound of formula (I) is 0.1-200 mg/ml, preferably 1-100 mg/ml, and more preferably 10-20 mg/ml;
the mass ratio of the compound of formula (I) to N,N-dimethylacetamide is 1:(0-100), preferably 1:(1-50), more preferably 1:(5-25), and further more preferably 1:(5-10);
the mass ratio of the compound of formula (I) to polyoxyethylene (35) castor oil is 1:(0-90), preferably 1:(5-50), more preferably 1:(15-40), further more preferably 1:(20-40), and even further more preferably 1:(25-40).

17. The injection preparation according to claim 15, wherein the content of the compound of formula (I) is 10 mg/ml.

18. The injection preparation according to claim 17, wherein the compound of formula (I) is at 20 mg, N,N-dimethylacetamide is at 100 mg, and polyoxyethylene (35) castor oil is at 800 mg.

19. A unit injection preparation, which is a concentrated ethanol solution labeled as having a volume of 2 ml and containing 20 mg of the compound of formula (I), 100 mg of N,N-dimethylacetamide and 800 mg of polyoxyethylene (35) castor oil.

20. An injection preparation, comprising the compound of formula (I) according to claim 1 and polyoxyethylene (35) castor oil.

21. An injection preparation, comprising the compound of formula (I) according to claim 1 and N,N-dimethylacetamide.

22. An injection preparation, comprising the compound of formula (I) according to claim 1, N,N-dimethylacetamide, and polyoxyethylene (35) castor oil.

23. The injection preparation according to any one of claims 1 to 22, wherein no water is added, and the percentage of water by mass is less than or equal to 1.0%, and preferably less than or equal to 0.5%.

24. An injection preparation product, comprising a packaging container and an injection preparation contained in the container, wherein the injection preparation is an injection preparation according to any one of the preceding claims 1 to 23 and is filled in a lightproof glass vial made of a neutral borosilicate glass tube, **characterized in that** the content of the compound of formula (I) in the injection preparation is 0.1-200 mg/ml, preferably 1-200 mg/ml, and further preferably 10-20 mg/ml.

25. An injection preparation product, which is an injection preparation that consists essentially of the compound of formula (I) according to claim 1, N,N-dimethylacetamide, polyoxyethylene (35) castor oil and ethanol, and is filled in a 2 ml-, 5 ml-, or 10 ml-lightproof glass vial made of a neutral borosilicate glass tube, **characterized in that** the content of the compound of formula (I) in the injection preparation is 10 mg/ml, the mass ratio of the compound of formula (I) to N,N-dimethylacetamide is 1:5, and the mass ratio of the compound of formula (I) to polyoxyethylene (35) castor oil is 1:40.

26. A method for preparing an injection preparation, comprising the following steps: adding a prescribed amount of the compound of formula (I) according to claim 1 to a prescribed amount of N,N-dimethylacetamide under stirring until dissolution, then adding a prescribed amount of polyoxyethylene (35) castor oil under stirring until dissolution, and finally adding ethanol until the prescribed volume for mixing and dissolving.

27. The method for preparing an injection preparation according to claim 26, **characterized in that** 20 mg of the compound of formula (I) according to claim 1 is added to 100 mg of N,N-dimethylacetamide and stirred until dissolution, then 800 mg of polyoxyethylene (35) castor oil is added and stirred until dissolution, and finally ethanol is added until a volume of 2ml for mixing and dissolving.

28. A method for preparing an injection preparation, comprising the following steps: adding a prescribed amount of the compound of formula (I) to a prescribed amount of N,N-dimethylacetamide under stirring until dissolution to obtain a first-dissolution preparation liquor; adding a prescribed amount of polyoxyethylene (35) castor oil to a portion of ethanol under stirring until dissolution to obtain a second-dissolution preparation liquor; and adding the first-dissolution preparation liquor to the second-dissolution preparation liquor under stirring until the mixed preparation is clear, and then adding ethanol until the prescribed volume for mixing and dissolving.

29. A ready-to-use injection for injection use, which is prepared from water, an isotonic regulator, and an injection preparation according to any one of claims 1 to 23, **characterized in that** the injection is added with or not added with a pH regulator.

30. A ready-to-use injection for injection use, comprising water, an isotonic regulator, the compound of formula (I) according to claim 1, N,N-dimethylacetamide, polyoxyethylene (35) castor oil, ethanol, and a pH regulator, **characterized in that** the injection is an isotonic solution with a pH value of 6.8-10.5, and the concentration of the compound of formula (I) is 0.016-1.10 mg/ml.

31. The ready-to-use injection for injection use according to claim 30, wherein the concentration of the compound of formula (I) is 0.020-1.00 mg/ml.

32. The ready-to-use injection for injection use according to claim 30, wherein the pH value of the injection for injection use is 7.4-8.1, and preferably 7.7.

33. The ready-to-use injection for injection use according to claim 29 or 30, wherein the pH regulator is selected from one or more of sodium citrate, potassium citrate, sodium acetate, potassium acetate, potassium dihydrogen phosphate, sodium dihydrogen phosphate, dipotassium hydrogen phosphate, disodium hydrogen phosphate, potassium phosphate, sodium phosphate, sodium hydroxide, potassium hydroxide, sodium bicarbonate, potassium bicarbonate, sodium carbonate, and potassium carbonate.

34. The ready-to-use injection for injection use according to claim 29 or 30, wherein the isotonic regulator is selected from anhydrous or hydrous sodium chloride, glucose, sucrose, fructose, xylitol, glycerol, sorbitol, mannitol, potassium chloride, mannose, calcium chloride, magnesium chloride, or a mixture thereof.

35. An injection for injection use, which consists of water, glucose as an isotonic regulator, the compound of formula (I) according to claim 1, N,N-dimethylacetamide, polyoxyethylene (35) castor oil, ethanol, and sodium bicarbonate as a pH regulator, **characterized in that** the injection for injection use is an isotonic solution with a pH value of 7.4, the concentration of the compound of formula (I) is 0.016-1.10 mg/ml, and preferably 0.020-1.00 mg/ml, and the mass percentage of the glucose content is 4.5-5.0%.

36. The injection for injection use according to claim 35, wherein the mass ratio of the compound of formula (I) to N,N-dimethylacetamide is 1:5, and the mass ratio of the compound of formula (I) to polyoxyethylene (35) castor oil is 1:40.

37. A method for preparing a ready-to-use injection for injection use, comprising the following steps: adding an appropriate amount of sodium bicarbonate solution as a pH regulator to a 5% glucose injection to obtain a mixed solution, such that the pH of the mixed solution is 7.7-8.1; and then adding an injection preparation according to any one of claims 1 to 23 or an injection preparation product according to claim 24 or 25 to the mixed solution for mixing and dissolving to obtain the ready-to-use injection for injection use.

38. The method for preparing a ready-to-use injection for injection use according to claim 37, wherein the mixed solution is obtained by adding 1 volume of a 5% sodium bicarbonate injection to 250 volumes of a 5% glucose injection.
